# EUROPEAN PATENT SPECIFICATION

(11) **EP 2 408 767 B1**
(45) Date of publication and mention of the grant of the patent: **10.07.2013**
(21) Application number: 10754142.7
(22) Date of filing: 19.03.2010
(51) Int. Cl.: C07D 413/12, C07D 413/10, C07D 413/06, A61P 25/28, A61K 31/535

(54) **AMIDE DERIVATIVES AS NEUROPEPTIDE Y5 RECEPTOR LIGANDS**
AMIDDERIVATE ALS NEUROPEPTID-Y5-REZEPTOR-LIGANDEN
DÉRIVÉS D'AMIDE EN TANT QUE LIGANDS DU RÉCEPTEUR DE NEUROPEPTIDE Y5

(30) Priority: 20.03.2009 US 161802 P
(43) Date of publication of application: 25.01.2012
(73) Proprietor: H. Lundbeck A/S, 2500 Valby-Copenhagen (DK)
(72) Inventor: CHEN, Bin, East Windsor NJ 08520 (US); BURNS, James, Ford, Glen Ridge NJ 07028 (US); DOLLER, Dario, Sparta NJ 07871 (US)
(74) Representative: Kalum, Bo
(86) International application number: PCT/US2010/027892
(87) International publication number: WO 2010/108052

(56) References cited:
- WO-A1-01/09120
- WO-A1-2004/089919
- US-B1- 6 380 224
- US-B1- 6 399 631

## Description

### FIELD OF THE INVENTION

The present invention is directed novel compounds which are ligands at the neuropeptide Y Y5 receptor. Separate aspects of the invention are directed to pharmaceutical compositions comprising said compounds and uses of the compounds to treat disorders related to mood, stress, cognition, stress and dementia.

### BACKGROUND ART

Neuropeptide Y (NPY) is a 36 amino acid neuropeptide expressed in the peripheral and central nervous system. This peptide is a member of the pancreatic polypeptide family, which also includes pancreatic polypeptide (PP) and peptide YY (PYY), and the biological effects ofNPY are mediated through its interaction with receptors that belong in the superfamily of G protein-coupled receptors.

Presently, five NPY receptor subtypes have been cloned: Y1 (D. Larhammar, et al., J. Biol. Chem., 1992, 267, 10935-10938); Y2 (C. Gerald, et al., J. Biol. Chem., 1995, 270, 26758-26761); Y4 (J. Bard, et al., J. Biol. Chem., 1995, 270, 26762-26765); Y5 (C. Gerald, et al., J. Biol. Chem., 1995, 270, 26758-26761); and y6 (P. Gregor, et al., J. Biol. Chem., 1996, 271, 27776-27781). All these receptor subtypes are expressed in several species except for the y6 subtype, which has been shown to be expressed in mouse and rabbit but not in rat and primate. A Y3 subtype has been proposed based on pharmacological data. However, the Y3 subtype has yet to be cloned and its existence remains to be fully established.

NPY exerts numerous physiological effects. On the basis of animal studies, it is evident that a contributory relationship exists between NPY and its receptors with disorders such as depression, anxiety and obesity. For instance, NPY expression is shown to be sensitive to energy status while NPY administration reduces energy expenditure. Another significant ability of NPY is to acutely stimulate feeding (S. Kalra, et al., Endocr. Rev., 1999, 20, 68-100). The NPY Y5 receptor has also been shown to be a receptor subtype responsible for NPY-induced food intake (C. Gerald, et al., Nature, 1996, 382, 168-171).

It is also reported that the NPY Y5 receptor mediates CNS effects in connection with stress and neuroendocrine disorders (M. Walker, et al. JPET, 2009, 328, 3. pg. 901-911). Lu AA33810, a selective Y5 receptor antagonist, attenuated increases in plasma ACTH and cortisol elicited by i.c.v. injection of cPP(1-7),NPY(19-23),Ala³¹,Aib³², Gln³⁴]. In Sprague Dawley rats subjected to the social interaction test, Lu AA33810 (3-30 mg/kg, p.o.) produced anxiolytic-like effects after acute or chronic treatment. In Flinders Sensitive Line rats, chronic dosing of Lu AA33810 (10 mg/kg/day, i.p.) produced anxiolytic-like effects in the social interaction test, plus antidepressant-like effects in the forced swim test. In Wistar rats exposed to chronic mild stress, chronic dosing of Lu AA33810 (3, 10 mg/kg/day, i.p.) produced antidepressant-like activity, i.e. normalization of stress-induced decrease in sucrose consumption.

PCT Publication No. WO 03/51397 claims the use of NPY Y5 receptor antagonists in the treatment of dementias as well the treatment of cognitive impairment disorders such as cognitive impairment associated with schizophrenia (CIAS). This application further discloses the use of NPY Y5 receptor antagonists to treat the positive and negative aspects of schizophrenia, autism, ADHD and Alzheimer's disease. The NPY Y5 receptor antagonist, MK-0557, is currently being tested in clinical trials for the treatment of cognitive impairment in patients with schizophrenia.

WO 2004/089919 describes cyaohexane carboxamido derivatives and their use as neuropeptide y receptor antagonists.

Although patients suffering from these disorders have available treatment options, many of these options lack the desired efficacy and are accompanied by undesired side effects. For example, the SSRIs have been a great advance for the treating mood disorders; however, many patients do not fully respond to treatments. Therefore, an unmet need exists for novel therapies for the treatment of said disorders.

In an attempt to identify new therapies, the inventors have identified a series of novel compounds as represented by Formula I and discovered that the compounds bind to the NPY Y5 receptor. Accordingly, the present invention provides novel compounds as medicaments for the treatment of disorders which are modulated by the NPY Y5 receptor.

### SUMMARY OF THE INVENTION

An objective of the present invention is to provide compounds that are ligands at the NPY Y5 receptor. Accordingly, the present invention relates to compounds of Formula I. 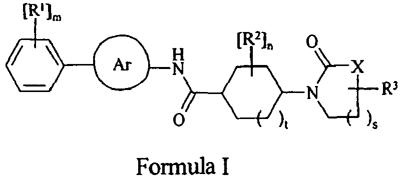 wherein R¹ is C₁-C₇ alkyl, C₁-C₇ perfluoroalkyl, C₁-C₇ alkoxy or halogen;
wherein R² is C₁-C₇ alkyl;
wherein R³ is H, C₁-C₇ alkyl, C₁-C₇ alkoxy, -(CH₂)ᵤOH, -N(R⁴)C(O)C₁-C₇ alkyl or - N(R⁴)C(O)C₁-C₇ alkoxy;
wherein R⁴ is H or C₁-C₇ alkyl;
wherein X is -CH₂-, -O-, -CH₂O-, -NR⁵- or -CH₂NR⁵-;
wherein R⁵ is H, C₁-C₇ alkyl, phenyl or pyridyl, where the phenyl and pyridyl are optionally substituted with C₁-C₇ alkyl, C₁-C₇ perfluoroalkyl, C₁-C₇ alkoxy or halogen;
wherein Ar is a divalent aromatic moiety selected from the group consisting of phenyl, pyridine, pyrimidine, pyridazine, pyrazine, imidazole, pyrazole, triazole, oxazole and isoxazole;
wherein each m and n is independently an integer from 0 to 5 inclusive;
wherein u is 0 or 1; and
wherein each t and s is independently an integer from 0 to 2 inclusive; or a pharmaceutically acceptable salt thereof.

In separate embodiments of the invention, the compound is selected from one of the specific compounds disclosed in the Experimental Section.

Furthermore, the present invention provides a pharmaceutical composition comprising a therapeutically effective amount of a compound of Formula I and a pharmaceutically acceptable carrier. The present invention also provides a process for making a pharmaceutical composition comprising admixing a therapeutically effective amount of a compound of Formula I and a pharmaceutically acceptable carrier.

The present invention provides a compound of formula I for use in a method of treating a subject suffering from depression. comprising administering to the subject a therapeutically effective amount of a compound of Formula I. Separately, the present invention further provides a method of treating a subject suffering from anxiety comprising administering to the subject a therapeutically effective amount of a compound of Formula I. The present invention further provides a method of treating a subject suffering from a cognitive disorder comprising administering to the subject a therapeutically effective amount of a compound of Formula I.

Additionally, the present invention is directed to the use of a compound as defined in Formula I for the manufacture of a medicament useful for treating depression. The present invention is directed to the use of a compound as defined in Formula I for the manufacture of a medicament useful for treating anxiety. The present invention further provides for the use of a compound as defined in Formula I for the manufacture of a medicament useful for treating cognitive disorders.

### DETAILED DESCRIPTION OF THE INVENTION

As previously indicated, the present invention is based on the discovery of the compound of Formula I are ligands at the at the NPY Y5 receptor, and as such, are useful for the treatment of related disorders. Additionally, certain aspects of the invention are explained in greater detail below but this description is not intended to be a detailed catalog of all the different ways in which the invention may be implemented, or all the features that may be added to the instant invention. Hence, the following specification is intended to illustrate some embodiments of the invention, and not to exhaustively specify all permutations, combinations and variations thereof.

As used herein, the term "C₁-C₇ alkyl" refers to a straight chained or branched saturated hydrocarbon having from one to seven carbon atoms inclusive. Examples of such substituents include, but are not limited to, methyl, ethyl, 1-propyl, 2-propyl, 1-butyl, 2-butyl, 2-methyl-2-propyl, 2-methyl-1-propyl, n-pentyl and n-hexyl. Similarly, the term "straight chained or branched C₁-C₄ alkyl" refers to a saturated hydrocarbon having from one to four carbon atoms inclusive. Examples of such substituents include, but are not limited to, methyl, ethyl and n-butyl.

Likewise, the term "C₁-C₇ alkoxy" refers to a straight chained or branched saturated alkoxy group having from one to seven carbon atoms inclusive with the open valency on the oxygen. Examples of such substituents include, but are not limited to, methoxy, ethoxy, n-butoxy, t-butoxy and n-heptyloxy.

As used herein, the term "C₁-C₇ perfluoroalkyl" refers to a straight chained or branched saturated hydrocarbon having from one to seven carbon atoms inclusive substituted with one or more fluorine atoms. Examples of such substituents include, but are not limited to, trifluoromethyl, pentafluoroethyl, 1-fluoroethyl, 1,2-difluoroethyl and 3,4 difluoroheptyl. Similarly, the term "straight chained or branched C₁-C₄ fluoroalkyl" refers to a saturated hydrocarbon having from one to four carbon atoms inclusive substituted with one or more fluorine atoms per carbon atom.

The term "halogen" refers to fluorine, chlorine, bromine and iodine.

As used herein, the term "mood disorders" includes major depressive disorder; minor depressive disorder; dysthymia; cyclothymia; bipolar depression; and depression NUD; and depressive obesity. Moreover, "major depressive disorder" is further divided into melancholic or atypical depression.

As used herein, the term "anxiety disorders" includes panic disorder; agoraphobia; social phobia (aka social anxiety disorder); obsessive compulsive disorder; and generalized anxiety disorder.

As used herein, the term "stress-related disorders" includes acute stress disorder, adjustment disorder; post traumatic stress disorder; exhaustion depression; and stress following (e.g. surgery and fever conditions).

As used herein, the term "sleep disorders" includes primary insomnia and disorders related to disturbances in circadian rhythms.

As used herein, the term "cognitive impairment/dysfunction" includes cognitive impairment associated with schizophrenia; dementias; autism; ADHD; and Alzheimer's disease. Moreover, "dementias" is further divided into age preceding dementia or AIDS dementia.

As used herein, the term "substance dependency/abuse" includes alcohol; nicotine; and cocaine addictions.

As used herein, the term "metabolic disorders" includes dyslipidemia; hyperlipidemia; insulin hyposensitivity; overweight/obesity; hyperglycemia; metabolic syndrome; and diabetes mellitus.

As used herein, the term "chronic pain disorders" include neuropathic pain; neuralgic pain; migraine; fibromyalgia; IBS; chronic fatigue syndrome; chronic tension type headache; chronic low back pain; myofascial pain and chronic osteoarthritis.

A "therapeutically effective amount" of a compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatment are two separate aspects of the invention. The patient to be treated is a mammal, in particular a human being.

Embodiments of the present invention are provided below.

In one embodiment, Ar is phenyl.

In a separate embodiment, Ar is pyridine or pyrimidine.

In one embodiment, Ar is pyridazine, pyrazine or triazole.

In one embodiment, Ar is imidazole, pyrazole, oxazole or isoxazole.

In one embodiment, t is 1 and where the cyclohexane moiety is in the cis configuration.

In one embodiment, t is 1 and where the cyclohexane moiety is in the trans configuration.

In one embodiment, X is -CH₂-.

In one embodiment, X is -O-.

In one embodiment, X is -CH₂O-.

In one embodiment, X is -NR⁵- and R⁵ is H or C₁-C₄ alkyl.

In one embodiment, X is -CH₂NR⁵-; and R⁵ is H or C₁-C₄ alkyl

In one embodiment, R¹ is C₁-C₄ alkoxy or C₁-C₄ perfluoroalkyl; and m is 0, 1 or 2.

In one embodiment, R¹ is C₁-C₄ alkyl, F or Cl; and m is 0, 1 or 2.

In one embodiment, n is 0.

In one embodiment, s is 0 or 1.

In one embodiment, R³ is H.

In one embodiment, R³ is C₁-C₄ alkyl, C₁-C₄ alkoxy or -(CH₂)ᵤOH; and u is 0.

In one embodiment, R³ is C₁-C₄ alkyl, C₁-C₄ alkoxy or -(CH₂)ᵤOH; and u is 1.

In one embodiment, R³ is -N(R⁴)C(O)C₁-C₄ alkyl, or -N(R⁴)C(O)C₁-C₄ alkoxy; and wherein R⁴ is H or C₁-C₄ alkyl.

In another embodiment, the compound is selected from the group consisting of trans-4-(2-Oxopyrrolidin-1-yl)-cyclohexanecarboxylic acid [5-(3, 5-difluoro-phenyl)-pyridin-2-yl]-amide; cis-4-(2-Oxo-pyrrolidin-1-yl)-cyclohexanecarboxylic acid [5-(3,5-difluoro-phenyl)-pyridin-2-yl]-amide; cis-4-(2-Oxo-pyrrolidin-1-yl)-cyclohexanecarboxylic acid [5-(3,5-dichloro-phenyl)-pyridin-2-yl]-amide; cis-4-(2-Oxo-pyrrolidin-1-yl)-cyclohexanecarboxylic acid [5-(3,5-difluoro-phenyl)-pyrazin-2-yl]-amide; 4-(2-Oxo-azeridin-1-yl)-cyclohexanecarboxylic acid [5-(3,5-difluorophenyl)-pyridin-2-yl]-amide; cis-4-(2-Oxo-piperidin-1-yl)-cyclohexanecarboxylic acid [5-(3,5-difluoro-phenyl)-pyridin-2-yl]-amide; cis-4-(2-Oxo-pyrrolidin-1-yl)-cyclohexanecarboxylic acid [5-(3-fluoro-5-methyl-phenyl)-pyridin-2-yl]-amide; cis-4-(2-Oxo-pyrrolidin-1-yl)-cyclohexanecarboxylic acid [5-(3-chloro-5-methyl-phenyl)-pyridin-2-yl]-amide; cis-4-(2-Oxo-oxazolidin-3-yl)-cyclohexanecarboxylic acid [5-(3,5-difluoro-phenyl)-pyridin-2-yl]-amide; trans-4-(2-Oxo-oxazolidin-3-yl)-cyclohexanecarboxylic acid [5-(3,5-difluoro-phenyl)-pyridin-2-yl]-amide; cis-4-(2-Oxo-oxazolidin-3-yl)-cyclohexanecarboxylic acid [5-(3,5-difluoro-phenyl)-pyrazin-2-yl]-amide; cis- 4-(2-Oxo-oxazolidin-3-yl)-cyclohexanecarboxylic acid [1-(3,5-difluorophenyl)-1H-pyrazol-3-yl]-amide; trans-4-(2-Oxo-[1,3]oxazinan-3-yl)-cyclohexanecarboxylic acid [1-(3,5-difluoro-phenyl)-1H-pyrazol-4-yl]-amide; cis-4-(2-Oxo-[1,3]oxazinan-3-yl)-cyclohexanecarboxylic acid [1-(3,5-difluoro-phenyl)-1H-pyrazol-4-yl]-amide; trans-4-(2-Oxo-[1,3]oxazinan-3-yl)-cyclohexanecarboxylic acid [1-(2-fluoro-phenyl)-1H-pyrazol-4-yl]-amide; cis-4-(2-Oxo-[1,3]oxazinan-3-yl)-cyclohexanecarboxylic acid [1-(2-fluoro-phenyl)-1H-pyrazol-4-yl]-amide; cis-4-(2-Oxo-[1,3]oxazinan-3-yl)-cyclohexanecarboxylic acid [5-(3,5-difluoro-phenyl)-pyrazin-2-yl]-amide; cis-4-(2-Oxo-tetrahydro-pyrimidin-1-yl)-cyclohexanecarboxylic acid [5-(3,5-difluoro-phenyl)-pyridin-2-yl]-amide; trans-4-(2-Oxo-tetrahydro-pyrimidin-1-yl)-cyclohexanecarboxylic acid [5-(3,5-difluoro-phenyl)-pyridin-2-yl]-amide; cis-4-(2-Oxo-imidazolidin-1-yl)-cyclohexanecarboxylic acid [5-(3,5-difluoro-phenyl)-pyridin-2-yl]-amide; cis-4-(3-Methyl-2-oxo-tetrahydro-pyrimidin-1-yl)-cyclohexanecarboxylic acid [5-(3,5-difluorophenyl)-pyridin-2-yl]-amide; cis-4-(3-Methyl-2-oxo-imidazolidin-1-yl)cyclohexanecarboxylic acid [5-(3,5-difluoro-phenyl)-pyridin-2-yl]-amide; trans-4-(3-Methyl-2-oxo-imidazolidin-1-yl)-cyclohexanecarboxylic acid [5-(3,5-difluoro-phenyl)-pyridin-2-yl]-amide; cis-4-(3-Oxo-morpholin-4-yl)-cyclohexanecarboxylic acid [5-(3,5-difluoro-phenyl)-pyridin-2-yl]-amide; trans-4-(3-Oxo-morpholin-4-yl)-cyclohexanecarboxylic acid [5-(3,5-difluoro-phenyl)-pyridin-2-yl]-amide; trans-4-(3-Oxo-morpholin-4-yl)-cyclohexanecarboxylic acid [1-(3,5-difluoro-phenyl)-1H-pyrazol-4-yl]-amide; cis-4-(3-Oxo-morpholin-4-yl)-cyclohexanecarboxylic acid [1-(3,5-difluorophenyl)-1H-pyrazol-4-yl]-amide; tans-4-(3-Oxo-morpholin-4-yl)-cyclohexanecarboxylic acid [1-(2-fluoro-phenyl)-1H-pyrazol-4-yl]-amide; cis-4-(3-Oxo-morpholin-4-yl)-cyclohexanecarboxylic acid [1-(2-fluoro-phenyl)-1H-pyrazol-4-yl]-amide; cis-4-(3-Oxo-morpholin-4-yl)-cyclohexanecarboxylic acid [5-(3,5-difluoro-phenyl)-pyrazin-2-yl]-amide; and cis-4-(3-Oxo-morpholin-4-yl)-cyclohexanecarboxylic acid [5-(3,5-difluoro-phenyl)-pyrimidin-2-yl]-amide; cis-4-(2-Oxo-pyrrolidin-1-yl)-cyclohexanecarboxylic acid [5-(3,5-difluoro-phenyl)-pyrimidin-2-yl]-amide; cis-4-(2-Oxo-oxazolidin-3-yl)-cyclohexanecarboxylic acid [5-(3,5-difluoro-phenyl)-pyrimidin-2-yl]-amide and cis-4-(3-Methyl-2-oxo-imidazolidin-1-yl)-cyclohexanecarboxylic acid [5-(3,5-difluoro-phenyl)-pyrimidin-2-yl]-amide.

In a separate embodiment, the compound is selected from one of the compounds disclosed in the Experimental Section.

### Pharmaceutically Acceptable Salts

The present invention also comprises salts of the present compounds, typically, pharmaceutically acceptable salts. Such salts include pharmaceutically acceptable acid addition salts. Acid addition salts include salts of inorganic acids as well as organic acids.

Representative examples of suitable inorganic acids include hydrochloric, hydrobromic, hydroiodic, phosphoric, sulfuric, sulfamic, nitric acids and the like. Representative examples of suitable organic acids include formic, acetic, trichloroacetic, trifluoroacetic, propionic, benzoic, cinnamic, citric, fumaric, glycolic, itaconic, lactic, methanesulfonic, maleic, malic, malonic, mandelic, oxalic, picric, pyruvic, salicylic, succinic, methane sulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, theophylline acetic acids, as well as the 8-halotheophyllines (for example, 8-bromotheophylline and the like). Further examples of pharmaceutically acceptable inorganic or organic acid addition salts include the pharmaceutically acceptable salts listed in S. M. Berge, et al., J. Pharm. Sci., 1977,66,2.

Furthermore, the compounds of this invention may exist in unsolvated as well as in solvated forms with pharmaceutically acceptable solvents such as water, ethanol and the like.

Racemic forms may be resolved into the optical antipodes by known methods, for example, by separation of diastereomeric salts thereof with an optically active acid, and liberating the optically active amine compound by treatment with a base. Separation of such diastereomeric salts can be achieved, e.g. by fractional crystallization. The optically active acids suitable for this purpose may include, but are not limited to d- or l- tartaric, mandelic or camphorsulfonic acids. Another method for resolving racemates into the optical antipodes is based upon chromatography on an optically active matrix. The compounds of the present invention may also be resolved by the formation and chromatographic separation of diastereomeric derivatives from chiral derivatizing reagents, such as, chiral alkylating or acylating reagents, followed by cleavage of the chiral auxiliary. Any of the above methods may be applied either to resolve the optical antipodes of the compounds of the invention per se or to resolve the optical antipodes of synthetic intermediates, which can then be converted by methods described herein into the optically resolved final products which are the compounds of the invention.

Additional methods for the resolution of optical isomers, known to those skilled in the art, may be used. Such methods include those discussed by J. Jaques, A. Collet and S. Wilen in Enantiomers, Racemates, and Resolutions, John Wiley and Sons, New York, 1981. Optically active compounds can also be prepared from optically active starting materials.

### Pharmaceutical compositions

The present invention further provides a pharmaceutical composition comprising a therapeutically effective amount of a compound of Formula I and a pharmaceutically acceptable carrier. The present invention also provides a pharmaceutical composition comprising a therapeutically effective amount of one of the specific compounds disclosed in the Experimental Section and a pharmaceutically acceptable carrier.

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington. The Science and Practice of Pharmacy, 19th Edition, Gennaro, Ed., Mack Publishing Co., Easton, PA, 1995.

The pharmaceutical compositions may be specifically formulated for administration by any suitable route such as oral, rectal, nasal, pulmonary, topical (including buccal and sublingual), transdermal, intracisternal, intraperitoneal, vaginal and parenteral (including subcutaneous, intramuscular, intrathecal, intravenous and intradermal) routes. It will be appreciated that the route will depend on the general condition and age of the subject to be treated, the nature of the condition to be treated and the active ingredient.

Pharmaceutical compositions for oral administration include solid dosage forms such as capsules, tablets, dragees, pills, lozenges, powders and granules. Where appropriate, the compositions may be prepared with coatings such as enteric coatings or they may be formulated so as to provide controlled release of the active ingredient such as sustained or prolonged release according to methods well known in the art. Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use.

Other suitable administration forms include, but are not limited to, suppositories, sprays, ointments, creams, gels, inhalants, dermal patches and implants.

Typical oral dosages range from about 0.001 to about 100 mg/kg body weight per day. Typical oral dosages also range from about 0.01 to about 50 mg/kg body weight per day. Typical oral dosages further range from about 0.05 to about 10 mg/kg body weight per day. Oral dosages are usually administered in one or more dosages, typically, one to three dosages per day. The exact dosage will depend upon the frequency and mode of administration, the sex, age, weight and general condition of the subject treated, the nature and severity of the condition treated and any concomitant diseases to be treated and other factors evident to those skilled in the art.

The formulations may also be presented in a unit dosage form by methods known to those skilled in the art. For illustrative purposes, a typical unit dosage form for oral administration may contain from about 0.01 to about 1000 mg, from about 0.05 to about 500 mg, or from about 0.5 to about 200 mg.

For parenteral routes such as intravenous, intrathecal, intramuscular and similar administration, typical doses are in the order of half the dose employed for oral administration.

The present invention also provides a process for making a pharmaceutical composition comprising admixing a therapeutically effective amount of a compound of Formula I and a pharmaceutically acceptable carrier. In an embodiment of the present invention the compound utilized in the aforementioned process is one of the specific compounds disclosed in the Experimental Section.

The compounds of this invention are generally utilized as the free substance or as a pharmaceutically acceptable salt thereof. One example is an acid addition salt of a compound having the utility of a free base. When a compound of Formula I contains a free base such salts are prepared in a conventional manner by treating a solution or suspension of a free base of Formula I with a molar equivalent of a pharmaceutically acceptable acid. Representative examples of suitable organic and inorganic acids are described above.

For parenteral administration, solutions of the compounds of Formula I in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The compounds of Formula I may be readily incorporated into known sterile aqueous media using standard techniques known to those skilled in the art.

Suitable pharmaceutical carriers include inert solid diluents or fillers, sterile aqueous solutions and various organic solvents. Examples of solid carriers include lactose, terra alba, sucrose, cyclodextrin, talc, gelatin, agar, pectin, acacia, magnesium stearate, stearic acid and lower alkyl ethers of cellulose. Examples of liquid carriers include, but are not limited to, syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining the compounds of Formula I and a pharmaceutically acceptable carrier are then readily administered in a variety of dosage forms suitable for the disclosed routes of administration. The formulations may conveniently be presented in unit dosage form by methods known in the art of pharmacy.

Formulations of the present invention suitable for oral administration may be presented as discrete units such as capsules or tablets, each containing a predetermined amount of the active ingredient, and optionally a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be tabletted, placed in a hard gelatin capsule in powder or pellet form or it may be in the form of a troche or lozenge. The amount of solid carrier will vary widely but will range from about 25 mg to about I g per dosage unit. If a liquid carrier is used, the preparation may be in the form of a syrup, emulsion, soft gelatin capsule or sterile injectable liquid such as an aqueous or non-aqueous liquid suspension or solution.

### Uses of the Compounds of Formula I

As mentioned above, the compounds of Formula I are ligands at the NPY Y5 receptor. The present invention provides a method of treating a subject suffering from a mood disorder which comprises administering to the subject a therapeutically effective amount of a compound of this invention. The present invention provides a method of treating a subject suffering from a cognitive disorder which comprises administering to the subject a therapeutically effective amount of a compound of this invention. This invention further provides a method of treating a subject suffering from obesity which comprises administering to the subject a therapeutically effective amount of a compound of this invention. In an embodiment of this invention, the subject is a human being.

Furthermore, the present invention is directed to the use of a compound of this invention for the manufacture of a medicament useful for treating depression. Additionally, the present invention is directed to the use of a compound of this invention for the manufacture of a medicament useful for treating anxiety. The present invention further provides for the use of a compound of a compound of this invention for the manufacture of a medicament useful for treating obesity.

The link between NPY and mood disorders such as depression and anxiety is established in the literature. For example, rats subjected to chronic mild stress exhibit anhedonia, a feature of clinical depression (P. Willner, et al., Eur. J. Pharmacol., 1997, 340, 121-132); they also contain elevated levels of NPY mRNA in hypothalamus accompanied by a reduction in hippocampus (V. Sergeyev, et al., Psychopharmacology, 2005, 178, 115-124). The behavioral changes associated with chronic mild stress are reversed by a variety of antidepressants (P. Willner, et al., Eur. J. Pharmacol., 1997, 340, 121-132). In one study of antidepressant therapies, rats treated with citalopram displayed an increased level of hippocampal NPY receptor binding with no change in NPY-like immunoreactivity (H. Husum, et al., Neuropsychopharmacology, 2001, 2, 183-191); conversely, electroconvulsive shock produced an increased level of hippocampal NPY-like immunoreactivity with no change in NPY receptor binding. These findings suggest that abnormal levels of NPY play a role in depressive illness, and that agents capable of regulating NPY and/or NPY receptor function particularly in limbic regions are useful for treating depression. Y5 is a NPY receptor expressed in limbic regions (M. Wolak, et al., J Comp. Neurol., 2003, 22, 285-311; and K. Nichol, et al., J. Neurosci., 1999, 19, 10295-10304). Accordingly, agents capable of regulating Y5 receptor function are therefore predicted to be useful for treating depression. Animal models of anxiety also reveal abnormal levels of NPY. In one example, maternally separated rats display an anxious and depressive phenotype throughout adulthood (R. Huot, Psychopharmacology, 2001, 158, 366-73); they also contain elevated levels of NPY-like immunoreactivity in hypothalamus accompanied by a reduction in hippocampus and cortex (P. Jimenez-Vasquez, Brain Res. Dev., 2001, 26, 149-152; H. Husum and A. Mathe, Neuropsychopharmacology, 2002 27:756-64; and H. Husum et al., Neurosci Lett., 2002, 333, 127-130). In a second example, rats subjected to fear conditioning display increased anxiety-like behavior; they also contain elevated levels of NPY in hypothalamus, amygdala and nucleus accumbens accompanied by a reduction in frontal cortex. The behavioral changes produced by fear conditioning can be reversed by treatment with anxiolytic drugs. In one study of fear conditioning, both the anxiety-like behavior and altered expression of NPY were reversed by treatment with diazepam (R. Krysiak, et al., Neuropeptides, 2000, 34, 148-57). These findings further suggest that NPY plays a role in anxiety, and that agents capable of regulating NPY and/or receptor function particularly in limbic regions are useful for treating anxiety. Y5 is a NPY receptor expressed in limbic regions (M. Wolak, et al., J. Comp. Neurol., 2003, 22, 285-311; and K. Nichol, et al., J. Neurosci., 1999, 19, 10295-10304). Thus, agents capable of regulating Y5 receptor function are therefore predicted to be useful for treating anxiety.

Several groups disclose the nexus between the NPY Y5 receptor and sleep disorders related to circadian rhythm disruptions. This nexus is based on the discovery that NPY Y5 receptors mediate an important physiologic response in the suprachiasmatic nucleus (SCN) of the hypothalamus in response to the application of NPY. For example, WO 99/05911 and WO 05/30208 disclose this link and propose the use of NPY Y5 receptor ligands for treating sleep disorders. Accordingly, it is expected that the compounds of Formula I can be used for treating sleep disorders, which includes primary insomnia, and disorders related to disturbances in circadian rhythms.

The pharmaceutical industry is also targeting NPY Y5 receptor antagonists as potential therapies for the treatment of cognitive impairment/dysfunction disorders. For example, the NPY Y5 receptor antagonist, MK-0557, is currently in clinical trials for the treatment of cognitive impairment in patients with schizophrenia. In support of this indication, WO 03/51356 proposes that NPY Y5 receptor antagonism can be used to treat dementias. Accordingly, it is expected that the compounds of Formula I can used for treating cognitive impairment/dysfunction disorders such as cognitive impairment associated with schizophrenia (CIAS). The compounds of the invention are also expected to treat the positive and negative aspects of schizophrenia; dementias; autism; ADHD; and Alzheimer's disease.

WO 02/28393 discloses methods of reducing self-administration of alcohol in a patient suffering from alcoholism comprising administering a NPY Y5 receptor antagonist. Accordingly, it is expected that the compounds of Formula I can used for treating substance dependency/abuse disorders such as alcoholism as well as nicotine and cocaine addictions.

Furthermore, it is expected that the compounds of Formula I can be used for treating metabolic disorders such as dyslipidemia; hyperlipidemia; insulin hyposensitivity; hyperglycemia; metabolic syndrome; and diabetes mellitus.

Analgesic-like effects of NPY have been shown in rats and mice both after intrathecal administration and ICV administration, and after infusion directly into specific brain regions. These studies have used either "spinal" pain models or "supraspinal" models. The involvement of the Y5 receptor subtype has been linked to chronic pain disorders (Woldbye, et al. Brain Research, 2007, 49-55). Thus, it is expected that the compounds-of Formula I can used for treating chronic pain disorders such as neuropathic pain; neuralgic pain; migraine; fibromyalgia; IBS; chronic fatigue syndrome; chronic tension type headache; chronic low back pain; myofascial pain and chronic osteoarthritis.

Additionally, it is expected the treatment of the cognitive and mood impairment in Parkinson's disease may be an indication target for the compounds of Formula I. The compounds of Formula I may also be used to treat disorders relating to and impulsivity are aggression.

The invention will be better understood from the Experimental Details which follow. However, one skilled in the art will readily appreciate that the specific methods and results discussed therein are merely illustrative of the invention as described more fully in the claims which follow thereafter. Furthermore, the variables depicted in Schemes 1-6 are consistent with the variables recited in the Summary of the Invention.

### EXPERIMENTAL SECTION

**General Methods:** Anhydrous solvents were purchased from the Aldrich Chemical Company and used as received. The NMR spectra were measured on a Bruker Avance 400 spectrometer and or 300 MHz (Varian) with CDCl₃, DMSO-d₆ or CD₃OD as the solvent. Chemical shifts (δ) are expressed in ppm, coupling constants (J) are expressed in Hz, and splitting patterns are described as follows: s=singlet; d=doublet; t=triplet; q=quartet; sept=septet; br=broad; m=multiplet; dd=doublet of doublets; dt=doublet of triplets; td=triplet of doublets; dq=doublet of quartet.

Unless otherwise noted, mass spectra were obtained using electrospray ionization (ESMS, Micromass Platform II or Quattro Micro) or Waters ZQ mass spectrometry with Agilent 1100 HPLC system with an autosampler using DAD/UV and Waters ELSD detection system and Inertsil ODS-3 column. For LC-MS determination, four methods were used: Method A: C18 column, Neutral pH, 20 % to 90 % Acetonitrile/ H₂O with 0.2 % Ammonium formate; or Method B: C18 column, Acidic pH, 0 % to 30 % Acetonitrile/ H₂O with 0.2 % Acetic acid; or Method C: C8 column, Neutral pH, 10 % to 90 % Acetonitrile/ H₂O with 0.2 % Ammonium formate; or Method D: C18 column, Neutral pH, 0 % to 30 % Acetonitrile/ H₂O with 0.2 % Ammonium formate.

In certain instances, the methods of preparing the compounds of the invention are described generally by referring to representative reagents such as bases or solvents. The particular reagent identified is representative but is not inclusive and does not limit the invention in any way.

It is noted that schemes 1-6 describe the use of selective protecting groups during the synthesis of the compounds of the invention. One skilled in the art would be able to select the appropriate protecting group for a particular reaction. Moreover, it may be necessary to incorporate protection and deprotection strategies for substituents such as amino, amido, carboxylic acid and hydroxyl groups in the synthetic methods described below to synthesize the compounds of Formula I. Methods for protection and deprotection of such groups are well known in the art, and may be found in T. Green, et al., Protective Groups in Organic Synthesis, 1991, 2nd Edition, John Wiley & Sons, New York.

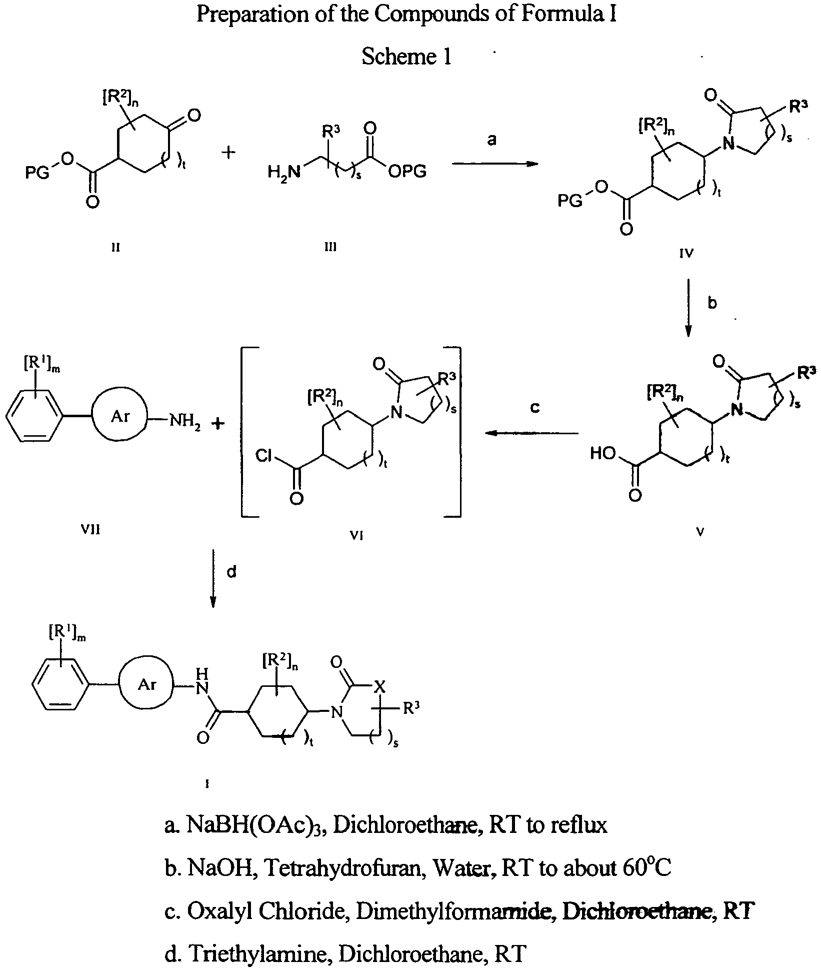

The compounds of Formula I wherein X is CH₂ and s is 0-2 may be synthesized according to the procedures described in Scheme 1. The starting materials of Formulas II and III are commercially available or may be synthesized by procedures known in the prior art. In summary, a keto ester of Formula II is combined with an amino ester of Formula III in the presence of a reducing agent to afford a lactam ester of Formula IV which is further hydrolyzed to form a lactam acid of Formula V. Conversion to an acid chloride of Formula VI followed by reaction with an amine of Formula VII affords the compounds of Formula I.

### Representative intermediates were synthesized according to Scheme 1.

### Intermediate of Formula IV, wherein R₃ is H and s is 1: 4-(2-Oxo-pyrrolidin-1-yl)-cyclohexanecarboxylic acid ethyl ester

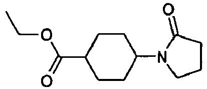

4-Oxo-cyclohexanecarboxylic acid ethyl ester (6.808g, 40.00mmoles) and 4-amino-butyric acid methyl ester hydrochloride (6.144g, 40.00mmoles) were dispersed with stirring in 1,2-dichloroethane (125mls). Sodium triacetoxyborohydride (16.96g, 80.00mmoles) was added in one portion and the reaction was heated to reflux overnight. The reaction mixture was diluted with 400ml ethyl acetate, transferred to a separatory funnel and washed with 150ml water followed by 100ml aqueous saturated sodium bicarbonate solution. The organic phase was dried over sodium sulfate, filtered and concentrated to afford the desired product as a mixture of cis and trans isomers which was used without further purification. ¹H NMR (400 MHz, CDCl₃) δ 4.1 (q, 2H), 3.9 (m, 1H), 3.3 (t, 2H), 2.60 (s, 1H), 2.30 (t, 2H), 2.15 (d, 2H), 2.05-1.70 (m, 3H), 1.60-1.30 (m, 5H), 1.2 (t,3H). ESI-MS m/z: 240 (M+H)⁺; t_{R} = 0.77min (Method A).

### Intermediate of Formula V, wherein R³ is H and s is 1: 4-(2-Oxo-pyrrolidin-1-yl)-cyclohexanecarboxylic acid

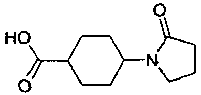

4-(2-Oxo-pyrrolidin-1-yl)-cyclohexanecarboxylic acid ethyl ester (9.5g, 40.00mmoles) was dissolved in tetrahydrofuran (200ml) and methanol (100ml). An aqueous solution of 1M sodium hydroxide (50ml, 50.00 mmoles) was added in one portion and the reaction was stirred at room temperature for 2 days. The reaction mixture was concentrated to ~50 ml and extracted with 300ml ethyl acetate. The aqueous phase was acidified to pH~1 with 1M aqueous hydrochloric acid and extracted 3 times with 200ml ethyl acetate. The combined organic phases were dried over sodium sulfate, filtered, and concentrated to afford the title compound as a mixture of cis and trans isomers. These were separated by mass directed prep HPLC [Column: Inertsil C18, 30 x 50 mm, 5 um particle size. Gradient: acetonitrile in water, 5-95% in 3 minutes with a cycle time of 5 min. A shallow gradient between 6-18% of acetonitrile was used between 0.51-2.5 min to separate cis and trans isomers. Flow rate: 100 mL/min. Mobile phase additive: 66 mM of acetic acid.]. cis isomer: 800mg, 9.5% yield: ¹H NMR (400MHz, CDCl₃) □4.00 (m, 1H), 3.35 (t, 2H), 2.73(s, 1H), 2.43 (t, 2H), 2.25 (m, 2H), 1.98 (m, 2H), 1.63 (m, 6H). ESI-MS m/z: 212 (M+H)⁺; t_{R} = 0.68 min (Method D). The cis stereochemistry was assigned based on the chemical shift (4.00ppm) and splitting (m) of the methine proton at C-1 of the cyclohexane ring and on the chemical shift (2.73ppm) and splitting (s) of the methine proton at C-4 of the cyclohexane ring in the NMR spectrum.
trans isomer: 1.7g, 20% yield: ¹H NMR (400MHz, CDCl₃) δ 3.98 (m, 1H), 3.35 (t, 2H), 2.40 (t, 2H), 2.25 (m, 1H), 2.12 (d, 2H), 2.03 (t, 2H) 1.80 (d, 2H), 1.60 (m, 2H), 1.45 (m, 2H). ESI-MS m/z: 212 (M+H)⁺; t_{R} = 0.76 min (Method D). The trans stereochemistry was assigned based on the chemical shift (3.98ppm) and splitting (m) of the methine proton at C-1 of the cyclohexane ring and on the chemical shift (2.25ppm) and splitting (m) of the methine proton at C-4 of the cyclohexane ring in the NMR spectrum.

### Compounds of the Invention

The compounds of Examples 1a -1h were prepared according to the procedures in Scheme 1.

### Example 1a trans-4-(2-Oxo-pyrrolidin-1-yl)-cyclohexanecarboxylic acid [5-(3,5-difluorophenyl)-pyridin-2-yl]-amide

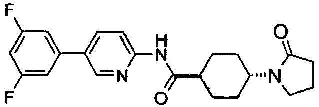

4-(2-Oxo-pyrrolidin-1-yl)-cyclohexanecarboxylic acid (211mg, 1mmole) was added to 1,2-dichloroethane (5ml) with stirring. Oxalyl chloride (150ul, 1.82 mmoles) followed by N,N-dimethylformamide (15ul, 0.19 mmoles). The reaction mixture was stirred for 3 hours at room temperature. The resulting oxo-(pyrrolidin-1-yl)-cyclohexanecarbonyl chloride solution was used without further purification.

5-(3,5-Difluoro-phenyl)-pyridin-2-ylamine (103mg, 0.50mmoles) and triethylamine (140uL, 1.00mmoles) were dissolved in dichloromethane (10ml). 4-(2-Oxo-pyrrolidin-1-yl)-cyclohexanecarbonyl chloride (115mg, 0.50mmoles) as a solution in dichloromethane (5mls) was added and the reaction mixture was stirred overnight at 55°C. After cooling to room temperature the reaction mixture was diluted with 20ml ethyl acetate, transferred to a separatory funnel, and washed with 10ml water. The organic phase was dried over sodium sulfate, filtered and concentrated in vacuo. The product was isolated by mass directed prep HPLC to yield 4 mg, 2% yield of the trans product as a white solid. ¹H NMR (400 MHz, CDCl₃) δ 8.39 (s, 1H), 8.26 (d, 1H), 8.00 (s, 1H), 7.81 (d, 1H), 7.00 (dd, 2H), 6.75 (tt, 1H), 3.96 (tt, 1H), 3 29 (t, 2H), 2.34 (t, 2H), 2.19 (tt, 1H), 2.06 (d, 2H), 1.95 (m, 2H), 1.81 (d, 2H), 1.69 (q, 2H), 1.46 (q, 2H). ESI-MS m/z: 400 (M+H)⁺; t_{R} = 1.17min (Method A). The trans stereochemistry was assigned based on the **chemical** shift (3.96ppm) and splitting (tt) of the methine proton at C-1 of the cyclohexane ring in the NMR spectrum.

Likewise, the following compounds were prepared analogously to that of Example 1 a:

### Example 1b cis-4-(2-Oxo-pyrrolidin-1-yl)-cyclohexanecarboxylic acid [5-(3,5-difluoro-phenyl)-pyridin-2-yl]-amide

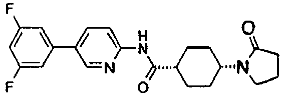

Prepared from cis-4-(2-oxo-pyrrolidin-1-yl)-cyclohexanecarboxylic acid and 5-(3,5-difluorophenyl)-pyridin-2-ylamine. ¹H NMR (400 MHz, CDCl₃) δ 8.48 (d, 1H), 8.35 (d, 1H), 8.19 (s, 1H), 7.91 (dd, 1H), 7.10 (d, 2H), 6.85 (tt, 1H), 4.07 (m, 1H), 3.38 (t, 2H), 2.73 (br s, 1H), 2.41 (t, 2H), 2.26 (d, 2H), 2.00 (m, 2H), 1.86 (m, 4H), 1.68 (d, 2H). ESI-MS m/z: 400 (M+H)⁺; t_{R} = 1.22min (Method A). The cis stereochemistry was assigned based on the chemical shift (4.07ppm) and splitting (m) of the methine proton at C-1 of the cyclohexane ring and on the chemical shift (2.73ppm) and splitting (br s) of the methine proton at C-4 of the cyclohexane ring in the NMR spectrum.

### Example 1c cis-4-(2-Oxo-pyrrolidin-1-yl)-cyclohexanecarboxylic acid [5-(3,5-dichloro-phenyl)-pyridin-2-yl]-amide

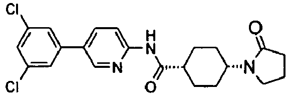

Prepared from cis-4-(2-oxo-pyrrolidin-1-yl)-cyclohexanecarboxylic acid and 5-(3,5-dichlorophenyl)-pyridin-2-ylamine. ¹H NMR (400 MHz, CDCl₃) δ 9.23 (s, 1H), 8.40 (d, 2H), 7.9 (d, 1H), 7.45-7.35 (3H), 4.04 (m, 1H), 3.38 (t, 2H), 2.74 (br s, 1H), 2.39 (t, 2H), 2.22 (d, 2H), 1.98 (m, 2H), 1.84 (m, 4H), 1.63 (d, 2H). ESI-MS m/z: 432 (M+H)⁺; t_{R} = 1.49min (Method A).

The cis stereochemistry was assigned based on the chemical shift (4.04ppm) and splitting (m) of the methine proton at C-1 of the cyclohexane ring and on the chemical shift (2.74ppm) and splitting (br s) of the methine proton at C-4 of the cyclohexane ring in the NMR spectrum.

### Example 1d cis-4-(2-Oxo-pyrrolidin-1-yl)-cyclohexanecarboxylic acid [5-(3,5-difluoro-phenyl)-pyrazin-2-yl]-amide

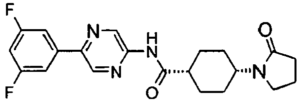

Prepared from cis-4-(2-oxo-pyrrolidin-1-yl)-cyclohexanecarboxylic acid and 5-(3,5-difluorophenyl)-pyrazin-2-ylamine. ¹H NMR (400 MHz, CDCl₃) δ 9.60 (s, 1H), 8.64 (s, 1H), 7.97 (s, 1H), 7.53 (d, 2H), 6.88 (t, 1H), 4.06 (m, 1H), 3.38 (t, 2H), 2.75 (s, 1H), 2.40 (t, 2H), 2.24 (d, 2H), 2.00 (m, 2H), 1.85 (m, 4H), 1.67 (br s, 2H). ESI-MS m/z: 401 (M+H)⁺; t_{R} = 1.20min (Method A). The cis stereochemistry was assigned based on the chemical shift (4.06ppm) and splitting (m) of the methine proton at C-1 of the cyclohexane ring and on the chemical shift (2.75ppm) and splitting (s) of the methine proton at C-4 of the cyclohexane ring in the NMR spectrum.

### Example 1e 4-(2-Oxo-azetidin-1-yl)-cyclohexanecarboxylic acid [5-(3,5-difluoro-phenyl)-pyridin-2-yl]-amide

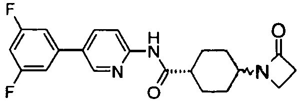

Prepared from 4-(2-oxo-azetidin-1-yl)-cyclohexanecarboxylic acid and 5-(3,5-difluoro-phenyl)-pyridin-2-ylamine. ¹H NMR (400 MHz, CDCl₃) δ 8.88 (br s, 1H), 8.49 (s, 1H), 8.28 (d, 1H), 7.86 (dd, 1H), 7.06 (dm, 2H), 6.81 (tt, 1H), 3.76 (t, 2H), 3.69 (br s, 1H), 2.78(t, 2H), 2.59 (br s, 1H), 1.80-1.59 (m, 8H). ESI-MS m/z: 386 (M+H)⁺; t_{R} = 1.16min (Method A).

### Example 1f cis-4-(2-Oxo-piperidin-1-yl)-cyclohexanecarboxylic acid [5-(3,5-difluoro-phenyl)-pyridin-2-yl]-amide

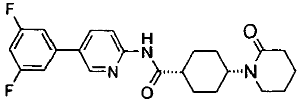

Prepared from cis-4-(2-oxo-piperidin-1-yl)-cyclohexanecarboxylic acid and 5-(3,5-difluorophenyl)-pyridin-2-ylamine. ¹H NMR (400 MHz, CDCl₃) δ 8.79 (br s, 1H), 8.45-8.38 (2H), 7.95 (dd, 1H), 7.06 (dm, 2H), 6.84 (tt, 1H), 4.59 (m, 1H), 3.18 (m, 2H), 2.73(m, 1H), 2.39 (m, 2H), 2.26 (d, 2H),1.92-1.76 (m, 4H), 1.73 (m, 4H), 1.59 (d, 2H). ESI-MS m/z: 414 (M+H)⁺; t_{R} = 1.31min (Method A). The cis stereochemistry was assigned based on the chemical shift (4.59ppm) and splitting (m) of the methine proton at C-1 of the cyclohexane ring and on the chemical shift (2.73ppm) and splitting (m) of the methine proton at C-4 of the cyclohexane ring in the NMR spectrum.

### Example 1g cis-4-(2-Oxo-pyrrolidin-1-yl)-cyclohexanecarboxylic acid [5-(3-fluoro-5-methylphenyl)-pyridin-2-yl]-amide

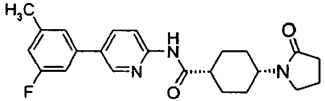

Prepared from cis-4-(2-oxo-pyrrolidin-1-yl)-cyclohexanecarboxylic acid and 5-(3-fluoro-5-methyl-phenyl)-pyridin-2-ylamine. ¹H NMR (400 MHz, CDCl₃) δ 8.46 (s, 1H), 8.29 (d, 1H), 8.21 (s, 1H), 7.88 (dd,1H), 7.13 (s, 1H), 7.04 (d, 1H), 6.89 (d, 1H), 4.03 (m, 1H), 3.36 (t, 2H), 2.69 (br s, 1H), 2.41 (s, 3H), 2.38 (t, 2H), 2.23 (d, 2H), 1.97 (m, 2H), 1.82 (m, 4H), 1.63 (m, 2H). ESI-MS m/z: 396 (M+H)⁺; t_{R} = 1.32min (Method C). The cis stereochemistry was assigned based on the chemical shift (4.03ppm) and splitting (m) of the methine proton at C-1 of the cyclohexane ring and on the chemical shift (2.69ppm) and splitting (br s) of the methine proton at C-4 of the cyclohexane ring in the NMR spectrum.

### Example 1h cis-4-(2-Oxo-pyrrolidin-1-yl)-cyclohexanecarboxylic acid [5-(3-chloro-5-methylphenyl)-pyridin-2-yl]-amide

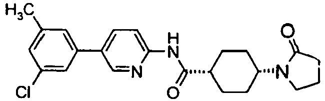

Prepared from cis-4-(2-oxo-pyrrolidin-1-yl)-cyclohexanecarboxylic acid and 5-(3-chloro-5-methyl-phenyl)-pyridin-2-ylamine. ¹H NMR (400 MHz, CDCl₃) δ 8.45 (d, 1H), 8.29 (d, 1H), 8.23 (s, 1H), 7.88 (dd, 1H), 7.33 (m, 1H), 7.22 (m, 1H), 7.18 (M, 1H), 4.08 (m, 1H), 3.36 (t, 2H), 2.69 (br s, 1H), 2.42-2.34 (5H), 2.23 (d, 2H), 1.97 (m, 2H), 1.91-1.72 (m, 4H), 1.68-1.60 (2H). ESI-MS m/z: 412 (M+H)⁺; t_{R} = 1.42min (Method A). The cis stereochemistry was assigned based on the chemical shift (4.08ppm) and splitting (m) of the methine proton at C-1 of the cyclohexane ring and on the chemical shift (2.69ppm) and splitting (br s) of the methine proton at C-4 of the cyclohexane ring in the NMR spectrum.

### Example 1i cis-4-(2-Oxo-pyrrolidin-1-yl)-cyclohexanecarboxylic acid [5-(3,5-difluoro-phenyl)-pyrimidin-2-yl]-amide

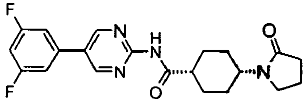

Prepared from cis-4-(2-oxo-pyrrolidin-1-yl)-cyclohexanecarboxylic acid and 5-(3, 5-difluorophenyl)-pyrimidin-2-ylamine. ESI-MS m/z: 401 (M+H]⁺; t_{R} = 0.92min (Method A).

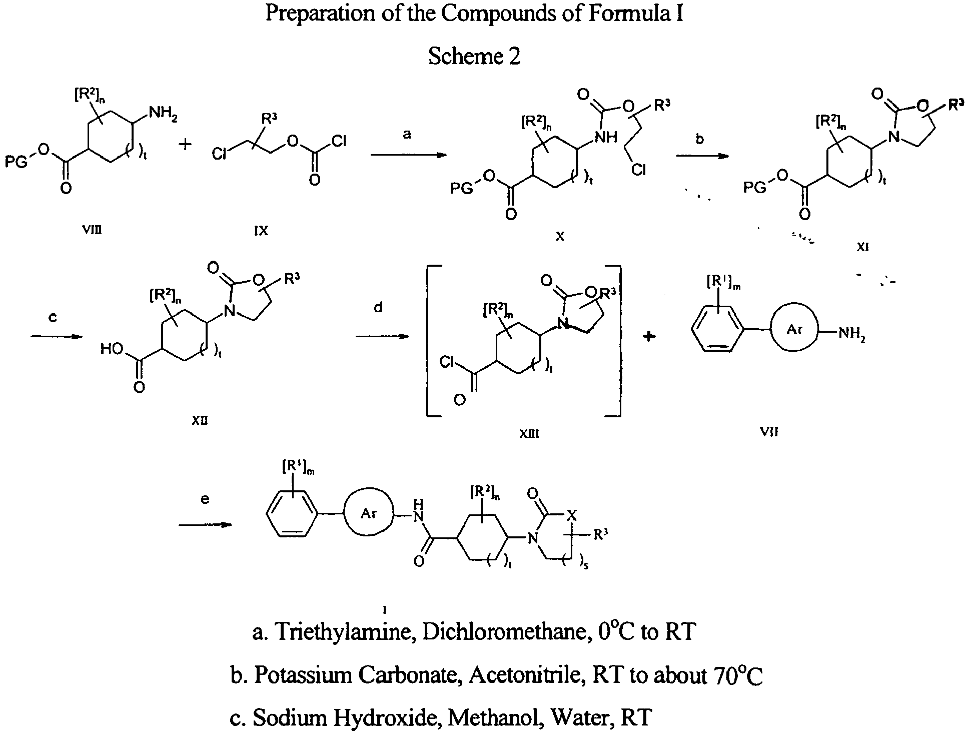

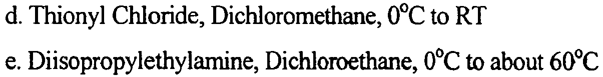

The compounds of Formula I, wherein X is O and s is 1, may be synthesized according to the procedures described in Scheme 2. The starting materials of Formulas VIII and IX are commercially available or may be synthesized by procedures known in the prior art. In summary, an amino ester of Formula VIII is combined with a chloroalkyl chloroformate of Formula IX to afford a chloroalkyl carbamate of Formula X which is further treated with a base to form an oxazolidinone ester acid of Formula XI. Hydrolysis to an acid of Formula XII followed by conversion to an acid chloride of Formula XIII followed by reaction with an amine of Formula VII affords the compounds of Formula I.

Representative intermediates were synthesized according to Scheme 2.

### Intermediate of Formula X, wherein R₃ is H: cis-4-(2-Chloro-ethoxycarbonylamino)-cyclohexanecarboxylic acid methyl ester

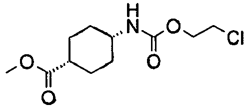

To a solution of cis-4-amino-cyclohexanecarboxylic acid methyl ester•HCl (1.93 g, 9.99 mmol) (Journal of Fluorine Chemistry, 1996, 80, 35-40) in methylene chloride (20mL) was added triethylamine (4.2mL, 30.1mmol), followed by the dropwise addition of a solution of 2-chloroethyl chloroformate (1.57g, 11.0mmol) in methylene chloride (5mL) at 0 °C. The mixture was stirred at rt for 3h. The reaction was quenched by the addition of saturated aqueous sodium bicarbonate (10ml). After stirring for 0.5h, the reaction mixture was transferred to a separatory funnel. The organic layer was separated, washed with brine, dried over MgSO₄ and concentrated in vacuo to give the crude product (2.60g, yield = 98%) which was used in the following reaction without further purification. ¹H NMR (400 MHz, CDCl₃) δ 4.83 (br s, 1H), 4.30 (t, 2H), 3.73-3.60 (m, 6H), 2.49 (m, 1H), 1.92-1.50 (m, 8H). ESI-MS m/z: 264.0 (M+H)⁺; t_{R} = 0.94min (Method A).

### Intermediate of Formula XI, wherein R₃ is H: cis-4-(2-Oxo-oxazolidin-3-yl)-cyclohexanecarboxylic acid methyl ester

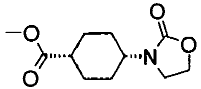

To a solution of cis-4-(2-chloro-ethoxycarbonylamino)-cyclohexanecarboxylic acid methyl ester (10.0g, 37.9mmol) in acetonitrile (200mL) was added potassium carbonate (10.5g, 76.0mmol). The reaction mixture was vigorously stirred at 70 °C for 24h. After cooling to rt, the reaction mixture was filtered and concentrated in vacuo. The residue was purified by flash chromatography (silica gel, gradient from 4/6 ethyl acetate/hexanes to 100% ethyl acetate) to give cis-4-(2-oxo-oxazolidin-3-yl)-cyclohexanecarboxylic acid methyl ester (5.66 g; Yield = 65.7%). ¹H NMR (400 MHz, CDCl₃): δ 4.30 (m, 2H), 3.72 (m, 1H), 3.70 (s, 3H), 3.50 (m, 2H), 2.65 (m, 1H), 2.24 (m, 2H), 1.75-1.50 (m, 6H). ESI-MS m/z: 227.9 (M+H)⁺; t_{R} = 0.58min (Method A).

### Intermediate of Formula XII, wherein R₃ is H: cis-4-(2-Oxo-oxazolidin-3-yl)-cyclohexanecarboxylic acid

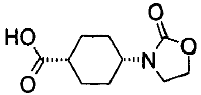

To a solution of 4-(2-oxo-oxazolidin-3-yl)-cyclohexanecarboxylic acid methyl ester (3.36g, 14.8mmol) in methanol (50mL) was added a solution of sodium hydroxide (1.25g, 31.3mmol) in water (15mL). The mixture was stirred at rt overnight. The reaction mixture was concentrated in vacuo. The residue was dissolved in 8.0mL water and the pH adjusted to ~4 by the addition of 12N HCl. The mixture was extracted with methylene chloride (5x10mL). The combined organic phases were dried over MgSO₄ and concentrated in vacuo to afford the crude product (3.0g, 95%) which was used without further purification. ¹H NMR (400 MHz, CDCl₃): δ 10.80 (br s, 1H), 4.33 (t, 2H), 3.72 (m, 1H), 3.53 (t, 2H), 2.75 (m, 1H), 2.29 (m, 2H), 1.77-1.61 (m, 6H). ESI-MS m/z: 213.9 (M+H)⁺. t_{R} = 0.97min (Method B).

### Compounds of the Invention

The compounds of Examples 2a - 2d were prepared according to the procedures in Scheme 2.

### Example 2a cis-4-(2-Oxo-oxazolidin-3-yl)-cyclohexanecarboxylic acid [5-(3,5-difluorophenyl)-pyridin-2-yl]-amide

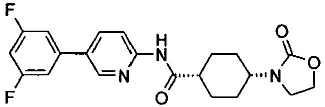

To a solution of cis-4-(2-oxo-oxazolidin-3-yl)-cyclohexanecarboxylic acid (3.40g, 15.9mmol) in methylene chloride (50mL) was added thionyl chloride (2.50mL, 34.3mmol) at room temperature. The mixture was stirred for 3h. The volatiles were removed in vacuo to afford the intermediate acid chloride. 5-(3,5-Difluoro-phenyl)-pyridin-2-ylamine (2.20g, 10.7mmol) was added to the acid chloride. 1,2-Dichloroethane (70mL) was added, followed by N,N-diisopropylethylamine (5.00mL, 28.7mmol). The reaction mixture was stirred at room temperature overnight. The resulting precipitate was collected by filtration and washed with a small amount of ethyl acetate. LC/MS showed it to be pure product (1.50g). The combined organic filtrate was transferred to a separatory funnel and washed with saturated aqueous NaHCO₃ followed by brine, dried over MgSO₄ and concentrated in vacuo. The resulting brown solid was recrystallized from DCM/EtOAc to give another 1.42g product. The mother liquor was concentrated and purified by MPLC using a solvent gradient from 30% ethyl acetate / 70% hexanes to 100% ethyl acetate to give a further 0.37 g of the desired product. ¹H NMR (400 MHz, CDCl₃): δ 8.49 (dd, 1H), 8.31 (dd, 1H), 8.06 (br s, 1H), 7.90 (dd, 1H), 7.10 (m, 2H), 6.85 (m, 1H), 4.34 (m, 2H), 3.81 (m, 1H), 3.57 (m, 2H), 2.71 (m, 1H), 2.26 (m, 2H), 1.95-1.75 (m, 6H). ESI-MS m/z: 401.9 (M+H)⁺; t_{R} = 1.20min (Method A).

Likewise, the following compounds were prepared analogously to that of Example 2a:

### Example 2b trans-4-(2-Oxo-oxazolidin-3-yl)-cyclohexanecarboxylic acid [5-(3,5-difluorophenyl)-pyridin-2-yl]-amide

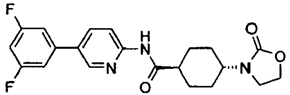

Prepared from trans-4-(2-oxo-oxazolidin-3-yl)-cyclohexanecarboxylic acid and 5-(3,5-difluorophenyl)-pyridin-2-ylamine. ¹H NMR (400 MHz, DMSO-d6) δ 10.63 (br s, 1H), 8.75 (m, 1H), 8.19 (m, 2H), 7.54 (m, 2H), 7.25 (m, 1H), 4.25 (m, 2H), 3.52 (m, 2H), 3.48 (m, 1H), 2.48 (m, 1H), 1.95 (m, 2H), 1.77 (m, 2H), 1.52 (m, 4H). ESI-MS m/z: 402.0 (M+H)⁺; t_{R} = 1.17min (Method A).

### Example 2c cis-4-(2-Oxo-oxazolidin-3-yl)-cyclohexanecarboxylic acid [5-(3,5-difluoro-phenyl)-pyrazin-2-yl]-amide

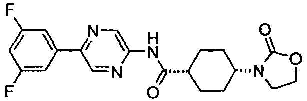

Prepared from cis-4-(2-oxo-oxazolidin-3-yl)-cyclohexanecarboxylic acid and 5-(3,5-difluorophenyl)-pyrazin-2-ylamine. ¹H NMR (400 MHz, CDCl₃) δ 9.60 (d, 1H), 8.66 (d, 1H), 8.01 (br s, 1H), 7.55 (m, 2H), 6.90 (m, 1H), 4.35 (m, 2H), 3.82 (m, 1H), 3.58 (m, 2H), 2.76 (m, 1H), 2.27 (m, 2H), 1.95-1.76 (m, 6H). ESI-MS m/z: 403.0 (M+H)⁺; t_{R} = 1.18min (Method A).

### Example 2d cis- 4-(2-Oxo-oxazolidin-3-yl)-cyclohexanecarboxylic acid [1-(3,5-difluoro-phenyl)-1H-pyrazol-3-yl]-amide

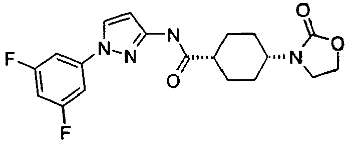

Prepared from cis-4-(2-oxo-oxazolidin-3-yl)-cyclohexanecarboxylic acid and 1-(3,5-difluorophenyl)-pyrazol-3-ylamine. ¹H NMR (400 MHz, CDCl₃) δ 7.94 (br s, 1H), 7.83 (d, 1H), 7.18 (m, 2H), 7.01 (d, 1H), 6.72 (m, 1H), 4.33 (m, 2H), 3.81 (m, 1H), 3.56 (m, 2H), 2.69 (m, 1H), 2.25 (m, 2H), 1.94-1.77 (m, 6H). ESI-MS m/z: 390.9 (M+H)⁺; t_{R} = 1.05min (Method A).

### Example 2e cis-4-(2-Oxo-oxazolidin-3-yl)-cyclohexanecarboxylic acid [5-(3,5-difluoro-phenyl)-pyrimidin-2-yl]-amide

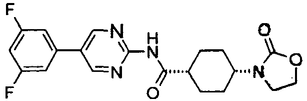

Prepared from cis-4-(2-oxo-oxazolidin-3-yl)-cyclohexanecarboxylic acid and 5-(3,5-difluorophenyl)-pyrimidin-2-ylamine. ESI-MS m/z: 402.9 (M+H)⁺; t_{R} = 0.89min (Method A).

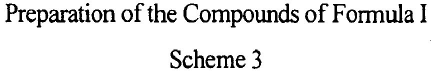

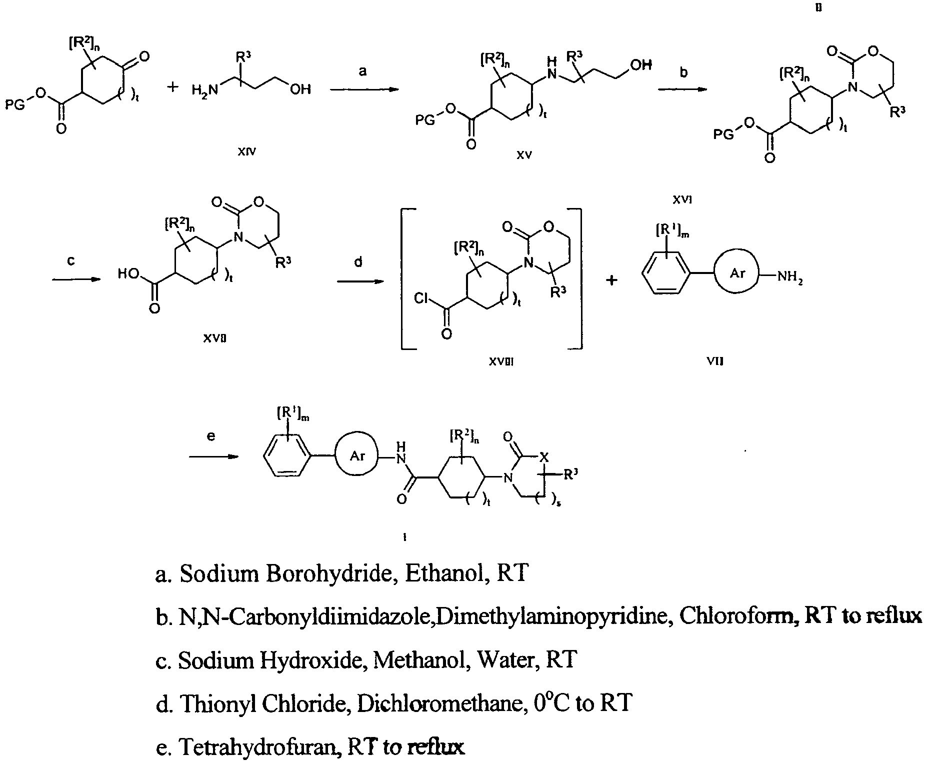

The compounds of Formula I, wherein X is O and s is 2, may be synthesized according to the procedures described in Scheme 3. The starting materials of Formulas II and XIV are commercially available or may be synthesized by procedures **known in** the **prior art. In summary,** an amino ester of Formula II is combined with an amino alcohol of Formula XIV in the presence of a reducing agent to afford an amino alcohol of Formula XV which is further treated with N,N-carbonyldiimidazole to form a cyclic carbamate ester of Formula XVI. Hydrolysis to an acid of Formula XVII followed by conversion to an acid chloride of Formula XIII followed by reaction with an amine of Formula VII affords the compounds of Formula I.

Representative intermediates were synthesized according to Scheme 3.

### Intermediate of Formula XV, wherein R₃ is H: 4-(3-Hydroxy-propylamino)-cyclohexanecarboxylic acid ethyl ester

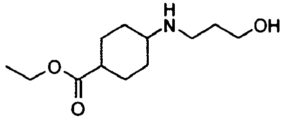

To a solution of 4-oxo-cyclohexanecarboxylic acid ethyl ester (3.85g, 22.6mmol) in ethanol (50mL) was added 3-amino-1-propanol (1.78g, 23.8mmol). The mixture was stirred at rt for 1h. Sodium tetrahydroborate (1.11g, 29.4mmol) was added and the resulting mixture was stirred at rt for 1h. The reaction was quenched by addition of 1.10mL water. Methylene chloride (60mL) was added to the mixture and the resulting white solid was removed by filtration. The filtrate was concentrated in vacuo to afford the crude product as a mixture of cis and trans isomers which was used without further purification. ¹H NMR (400 MHz, CDCl₃) δ 4.14-4.07 (m, 2H), 3.82 (m, 2H), 2.89 (m, 2H), 2.70-1.00 (m, 17H). ESI-MS m/z: 230.1 (M+H)⁺; t_{R} = 0.58min (Method B).

### Intermediate of Formula XVI, wherein R₃ is H: 4-(2-Oxo-perhydro-1,3-oxazin-3-yl)-cyclohexanecarboxylic acid ethyl ester

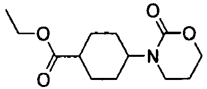

To a solution of 4-(3-hydroxy-propylamino)-cyclohexanecarboxylic acid ethyl ester (2.82g, 12.3mmol) in chloroform (60mL) was added 4-dimethylaminopyridine (15.0mg, 0.123mmol) and N,N-carbonyldiimidazole (2.09g, 12.9mmol). The reaction was stirred at rt for 2h, then refluxed for 18h. After cooling to rt the mixture was transferred to a separatory funnel and washed with saturated NH₄Cl then brine. The organic phase was dried over MgSO₄ and concentrated in vacuo to afford the crude product as a mixture of cis and trans isomers which was used without further purification. ¹H NMR (400 MHz, CDCl₃) δ 4.25-4.15 (m, 5H), 3.21 (m, 2H), 2.80-1.10 (m, 14H). ESI-MS m/z: 256.0 (M+H)⁺; t_{R} = 1.65min. ESI-MS m/z: 256.0 (M+H)⁺; t_{R} = 1.74min (Method B).

### Intermediate of Formula XVII, wherein R₃ is H: 4-(2-Oxo-perhydro-1,3-oxazin-3-yl)-cyclohexanecarboxylic acid

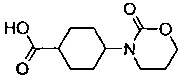

To a solution of 4-(2-oxo-perhydro-1,3-oxazin-3-yl)-cyclohexanecarboxylic acid ethyl ester (2.95g, 11.6mmol) in methanol (50mL) was added a solution of sodium hydroxide (0.924g, 23.1mmol) in water (15mL). The reaction mixture was stirred at rt overnight. The solvents were removed in vacuo. The residue was dissolved in 10mL water and the pH adjusted to ~4 by addition of 12N HCl. The mixture was extracted with CHCl₃/i-PrOH=3/1. The combined organic phases were dried over MgSO₄ and concentrated in vacuo to afford the crude product as a mixture of cis and trans isomers which was used without further purification. ¹H NMR (400 MHz, CDCl₃) δ 9.50 (br s, 1H), 4.22 (m, 2H), 4.14 (m, 1H), 3.21 (m, 2H), 2.72-1.10 (m, 11H). ESI-MS m/z: 228.0 (M+H)⁺; t_{R}=0.94min. ESI-MS m/z: 228.0 (M+H)⁺; t_{R} = 1.01min (Method B).

### Compounds of the Invention

The compounds of Examples 3a- 3g were prepared according to the procedures in Scheme 3.

### Examples 3a and 3b cis-4-(2-Oxo-perhydro-1,3-oxazin-3-yl)-cyclohexanecarboxylic acid [5-(3,5-difluoro-phenyl)-pyridin-2-yl]-amide and trans-4-(2-Oxo-perhydro-1,3-oxazin-3-yl)-cyclohexanecarboxylic acid [5-(3,5-difluoro-phenyl)-pyridin-2-yl]-amide

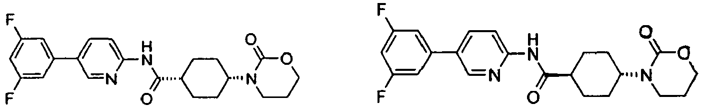

To a solution of 4-(2-oxo-perhydro-1,3-oxazin-3-yl)-cyclohexanecarboxylic acid (150mg, 0.66mmol) in methylene chloride (10mL) was added thionyl chloride (0.10mL, 1.37mmol). The reaction mixture was stirred at room temperature for 3h. The volatiles were removed in vacuo. The residue was dissolved in tetrahydrofuran (20mL) and 5-(3,5-difluoro-phenyl)-pyridin-2-ylamine (136mg, 0.660mmol) was added. The reaction mixture was refluxed for 18h. After cooling to room temperature the reaction mixture was transferred to a separatory funnel and was washed with saturated NaHCO₃ then brine. The organic phase was dried over MgSO₄ and concentrated in vacuo. The product mixture was purified by MPLC using a solvent gradient from 30% ethyl acetate / 70% hexanes to 100% ethyl acetate to afford the desired products. (cis product 28 mg; Yield =10%.) ¹H NMR (400 MHz, CDCl₃): δ 8.40 (dd, 1H), 8.24 (d, 1H), 8.02 (br s, 1H), 7.90 (dd, 1H), 7.01 (m, 2H), 6.76 (m, 1H), 4.21 (m, 1H), 4.15 (m, 2H), 3.18 (m, 2H), 2.63 (m, 1H), 2.18 (m, 2H), 1.95-1.60 (m, 8H). ESI-MS m/z: 415.9 (M+H)⁺; t_{R} = 1.17min (Method A). The cis stereochemistry was assigned based on the chemical shift (4.21ppm) and splitting (m) of the methine proton at C-1 of the cyclohexane ring and on the chemical shift (2.63ppm) and splitting (m) of the methine proton at C-4 of the cyclohexane ring in the NMR spectrum.
(trans product 25mg, Yield = 9%). ¹H NMR (400 MHz, DMSO-d6): δ 10.63 (br s, 1H); 8.75 (m, 1H), 8.19 (d, 2H), 7.54 (m, 2H), 7.25 (m, 1H), 4.21 (t, 2H), 3.86 (m, 1H), 3.20 (t, 2H), 2.47 (m, 1H), 1.97-1.88 (m, 4H), 1.69 (m, 2H), 1.54 (m, 4H). ESI-MS m/z: 415.9 (M+H)⁺; t_{R} = 1.13min (Method A). The trans stereochemistry was assigned based on the chemical shift (3.86ppm) and splitting (m) of the methine proton at C-1 of the cyclohexane ring and on the chemical shift (2.47ppm) and splitting (m) of the methine proton at C-4 of the cyclohexane ring in the NMR spectrum.

Likewise, the following compounds were prepared analogously to that of Examples 3a and 3b:

### Example 3c trans-4-(2-Oxo-[1,3]oxazinan-3-yl)-cyclohexanecarboxylic acid [1-(3,5-difluorophenyl)-1H-pyrazol-4-yl]-amide

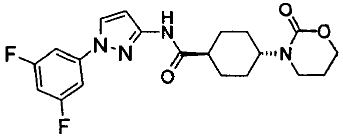

Prepared from trans-4-(2-oxo-perhydro-1,3-oxazin-3-yl)-cyclohexanecarboxylic acid and 1-(3,5-difluoro-phenyl)-1H-pyrazol-3-ylamine. ¹H NMR (400 MHz, CDCl₃): δ 7.85 (br s, 1H), 7.81 (d, 1H), 7.17 (m, 2H), 7.02 (m, 1H), 6.71 (m, 1H), 4.25 (t, 2H), 4.16 (m, 1H), 3.27 (t, 2H), 2.31-1.53 (m, 11H). ESI-MS m/z: 404.9 (M+H)⁺; t_{R} = 1.01min (Method A).

### Example 3d cis-4-(2-Oxo-[1,3]oxazinan-3-yl)-cyclohexanecarboxylic acid [1-(3,5-difluorophenyl)-1H-pyrazol-4-yl]-amide

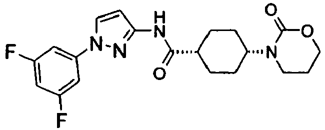

Prepared from cis-4-(2-oxo-perhydro-1,3-oxazin-3-yl)-cyclohexanecarboxylic acid and 1-(3,5-difluoro-phenyl)-1H-pyrazol-3-ylamine. ¹H NMR (400 MHz, CDCl₃): δ 7.92 (br s, 1H), 7.83 (d, 1H), 7.18 (m, 2H), 7.01 (d, 1H), 6.72 (m, 1H), 4.29 (m, 1H), 4.23 (m, 2H), 3.26 (m, 2H), 2.69 (m, 1H), 2.25 (m, 2H), 2.04-1.69 (m, 8H). ESI-MS m/z: 404.9 (M+H)⁺; t_{R} = 1.04min (Method A).

### Example 3e trans-4-(2-Oxo-[1,3]oxazinan-3-yl)-cyclohexanecarboxylic acid [1-(2-fluorophenyl)-1H-pyrazol-yl]-amide

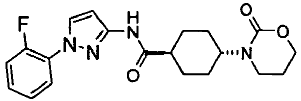

Prepared from trans-4-(2-oxo-perhydro-1,3-oxazin-3-yl)-cyclohexanecarboxylic acid and 1-(2-fluoro-phenyl)-1H-pyrazol-3-ylamine. ¹H NMR (400 MHz, CDCl₃): δ 8.09 (br s, 1H), 7.92 (t, 1H), 7.77 (m, 1H), 7.26 (m, 3H), 7.00 (d, 1H), 4.25 (t, 2H), 4.15 (m, 1H), 3.26 (t, 2H), 2.26-1.49 (m, 11H). ESI-MS m/z: 386.9 (M+H)⁺; t_{R} = 0.88min (Method A).

### Example 3f cis-4-(2-Oxo-[1,3]oxazinan-3-yl)-cyclohexanecarboxylic acid [1-(2-fluoro-phenyl)-1H-pyrazol-4-yl]-amide

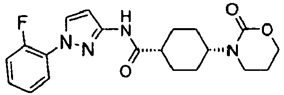

Prepared from cis-4-(2-oxo-perhydro-1,3-oxazin-3-yl)-cyclohexanecarboxylic acid and 1-(2-fluoro-phenyl)-1H-pyrazol-3-ylamine. ¹H NMR (400 MHz, CDCl₃): δ 8.09 (br s, 1H), 7.94 (t, 1H), 7.78 (m, 1H), 7.26 (m, 3H), 7.00 (d, 1H), 4.30 (m, 1H), 4.23 (m, 2H), 3.26 (t, 2H), 2.69 (m, 1H), 2.24 (m, 2H), 2.04-1.66 (m, 8H). ESI-MS m/z: 386.9 (M+H)⁺; t_{R} = 0.93min (Method A).

### Example 3g cis-4-(2-Oxo-[1,3]oxazinan-3-yl)-cyclohexanecarboxylic acid [5-(3,5-difluorophenyl)-pyrazin-2-yl]-amide

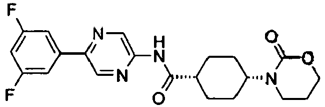

Prepared from cis-4-(2-oxo-perhydro-1,3-oxazin-3-yl)-cyclohexanecarboxylic acid and 5-(3,5-difluoro-phenyl)-pyrazin-2-yl-amine. ¹H NMR (400 MHz, CDCl₃): δ 9.60 (d, 1H), 8.65 (d, 1H), 7.97 (br s, 1H), 7.55(m, 2H), 6.90 (m, 1H), 4.31 (m, 1H), 4.24 (t, 2H), 3.27 (t, 2H), 2.76 (m, 1H), 2.27 (m, 2H), 2.06-1.69 (m, 8H). ESI-MS m/z: 416.9 (M+H)⁺; t_{R} = 1.19min (Method A).

### Preparation of the Compounds of Formula I

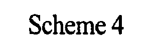

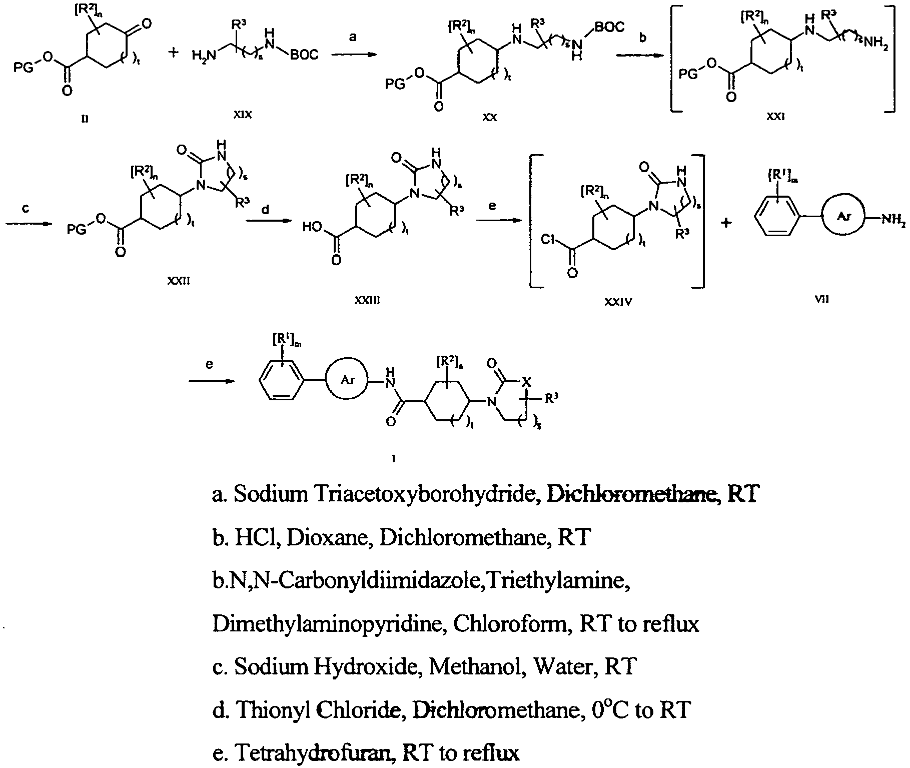

The compounds of Formula I wherein X is NH and s is 1 or 2 may be synthesized according to the procedures described in Scheme 4. The starting materials of Formulas II and XIX are commercially available or may be synthesized by procedures known in the prior art. In summary, a keto ester of Formula II is combined with a mono-protected diamine of Formula XIX in the presence of a reducing agent to afford a mono-protected diamine of Formula XX. This is deprotected to a diamine of Formula XXI which is further treated with N,N-carbonyldiimidazole to form a cyclic urea ester of Formula XXII. Hydrolysis to an acid of Formula XXIII followed by conversion to an acid chloride of Formula XXIV followed by reaction with an amine of Formula VII affords the compounds of Formula I.

Representative intermediates were synthesized according to Scheme 4.

### Intermediate of Formula XX, wherein R₃ is H and s is 2: 4-(3-tert-Butoxycarbonylamino-propylamino)-cyclohexanecarboxylic acid ethyl ester

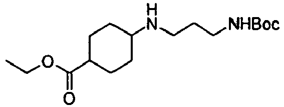

To a solution of 4-oxo-cyclohexanecarboxylic acid ethyl ester (2.50 g, 14.7 mmol) in methylene chloride (60mL) was added N-(3-aminopropyl)(tert-butoxy)carboxamide (2.60g, 14.9mmol). The mixture was stirred for 1h at rt. Sodium triacetoxyborohydride (5.0g, 23.6nmol) was then added and the resulting mixture was stirred at rt for 4h. The reaction mixture was quenched by addition of saturated NH₄Cl. The phases were separated and the aqueous phase was extracted with methylene chloride. The combined organic phases were concentrated in vacuo to afford the crude product as a mixture of cis and trans isomers (4.71g; Yield = 97.6%) which was used without further purification. ¹H NMR (400 MHz, CDCl₃) δ 5.37 (m, 1H), 4.21-4.10 (m, 2H), 3.29 (m, 2H), 3.13-2.90 (m, 3H), 2.65-1.50 (m, 12H), 1.44 (s, 9H), 1.31-1.26 (m, 3H). ESI-MS m/z: 329.0 (M-+H)⁺; t_{R} = 1.12min (Method B).

### Intermediate of Formula XXII, wherein R₃ is H and s is 2: 4-(2-Oxo-tetrahydro-pyrimidin-1-yl)-cyclohexanecarboxylic acid ethyl ester

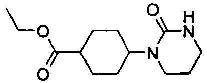

To a solution of 4-(3-tert-butoxycarbonylamino-propylamino)-cyclohexanecarboxylic acid ethyl ester (4.70g, 14.3mmol) in methylene chloride (50mL) was added 4M of hydrogen chloride in 1,4-dioxane (20mL). The mixture was stirred at room temperature for 3h. The volatiles were removed in vacuo to afford the crude product 4-(3-amino-propylamino)-cyclohexanecarboxylic acid ethyl ester•2[HCl] (4.30g; Yield = 99.7%) as a mixture of cis and trans isomers. ESI-MS m/z: 229.1 (M+H)⁺; t_{R}= 0.27min (Method B).

This intermediate (4.30g, 14.3mmol) was dissolved in chloroform (100mL) and triethylamine (5.0mL, 35.9mmol), 4-dimethylaminopyridine (20mg, 0.164mmol) and N,N-carbonyldiimidazole (2.43g, 15.0mmol) were added. After stirring at rt for 1h, the mixture was refluxed for 18h. After cooling to rt, the reaction mixture was transferred to a separatory funnel and washed with saturated NH₄Cl followed by brine. The organic phase was dried over MgSO₄ and concentrated in vacuo to afford the crude product as a mixture of cis and trans isomers which was used without further purification. ¹H NMR (400 MHz, CDCl₃) δ 4.70-4.60 (m; 1H), 4.31 (m, 1H), 4.21-4.10 (m, 2H), 3.27 (m, 2H), 3.20-3.10 (m, 2H), 2.70-1.43 (m, 11H), 1.32-1.24 (m, 3H). ESI-MS m/z: 255.0 (M+H)⁺; t_{R}=1.66min (Method B).

### Intermediate of Formula XXIII, wherein R₃ is H and s is 2: 4-(2-Oxo-tetrahydro-pyrimidin-1-yl)-cyclohexanecarboxylic acid

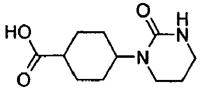

To a solution of 4-(2-oxo-tetrahydro-pyrimidin-1-yl)-cyclohexanecarboxylic acid ethyl ester (1.00g, 3.93mmol) in methanol (30mL) was added a solution of sodium hydroxide (0.50g, 12mmol) in water (6mL). The mixture was stirred at room temperature overnight. The volatiles were removed in vacuo. The residue was dissolved in 6mL water and the pH adjusted to ∼3 by addition of 12N HCl. The mixture was transferred to a separatory funnel and extracted with dichloromethane (5x5mL). The combined organic phases were dried over MgSO₄ and concentrated in vacuo to afford the crude product as a mixture of cis and trans isomers which was used in the following reactions without further purification. ¹H NMR (400 MHz, DMSO-d6) δ 12.13 (br s, 1H), 6.12 (m, 1H), 4.06 (m, 1H), 3.10-2.98 (m, 4H), 2.56-1.30 (m, 11H). ESI-MS m/z: 227.1 (M+H)⁺; t_{R} = 0.93min, ESI-MS m/z: 227.1 (M+H)⁺; t_{R} =1.09min (Method B).

### Compounds of the Invention

The compounds of Examples 4a-4c were prepared according to the procedures in Scheme 4.

### Examples 4a and 4b cis-4-(2-Oxo-tetrahydro-pyrimidin-1-yl)-cyclohexanecarboxylic acid [5-(3,5-difluoro-phenyl)-pyridin-2-yl]-amide and trans-4-(2-Oxo-tetrahydro-pyrimidin-1-yl)-cyclohexanecarboxylic acid [5-(3,5-difluoro-phenyl)-pyridin-2-yl]-amide

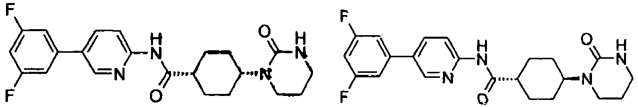

To a solution of 4-(2-oxo-tetrahydro-pyrimidin-1-yl)-cyclohexanecaboxylic acid (149mg, 0.66mmol) in methylene chloride (10mL) was added thionyl chloride (0.1mL, 1.37mmol) at rt. The reaction mixture was stirred at rt for 3h. The volatiles were removed in vacuo. The residue was dissolved in tetrahydrofuran (20mL). 5-(3,5-Difluoro-phenyl)-pyridin-2-ylamine (136mg, 0.66mmol) was added and the reaction mixture was refluxed overnight. After cooling to room temperature the reaction mixture was transferred to a separatory funnel and washed with saturated NaHCO₃ followed by brine. The organic phase was dried over MgSO₄ and concentrated in vacuo. The residue was purified by MPLC using a solvent gradient from 30% ethyl acetate / 70% hexanes to 100% ethyl acetate to give the desired products. cis product 22 mg, ¹H NMR (400 MHz, CDCl₃) δ 8.48 (dd, 1H), 8.37 (br s, 1H), 8.35 (dd, 1H), 7.90 (dd, 1H), 7.10 (m, 2H), 6.85 (m, 1H), 4.77 (br s, 1H), 4.41 (m, 1H), 3.28 (m, 2H), 3.22 (m, 2H), 2.73 (m, 1H), 2.26 (m, 2H), 1.92-1.78 (m, 6H), 1.65 (m, 2H). ESI-MS m/z: 415.0 (M+H)⁺; t_{R} = 1.16min (Method A). The cis stereochemistry was assigned based on the chemical shift (4.41ppm) and splitting (m) of the methine proton at C-1 of the cyclohexane ring and on the chemical shift (2.73ppm) and splitting (m) of the methine proton at C-4 of the cyclohexane ring in the NMR spectrum.
trans product 23mg, ¹H NMR (400 MHz, DMSO-d6) δ 10.62 (br s, 1H), 8.75 (m, 1H), 8.19 (s, 2H), 7.55 (d, 2H), 7.25 (m, 1H), 6.15 (s, 1H), 4.08 (m, 1H), 3.11 (m, 2H), 3.07 (m, 2H), 2.45 (m, 1H), 1.91 (m, 2H), 1.76 (m, 2H), 1.61-1.45 (m, 6H). ESI-MS m/z: 415.0 (M+H)⁺; t_{R} = 1.11min (Method A). The trans stereochemistry was assigned based on the chemical shift (4.08ppm) and splitting (m) of the methine proton at C-1 of the cyclohexane ring and on the chemical shift (2.45ppm) and splitting (m) of the methine proton at C-4 of the cyclohexane ring in the NMR spectrum.

Likewise, the following compounds were prepared analogously to that of Examples 4a and 4b:

### Examples 4c cis-4-(2-Oxo-imidazolidin-1-yl)-cyclohexanecarboxylic acid [5-(3,5-difluorophenyl)-pyridin-2-yl]-amide

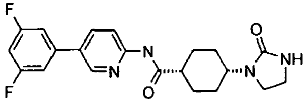

Prepared from cis-4-(2-Oxo-imidazolidin-1-yl)-cyclohexanecarboxylic acid and 5-(3,5-difluorophenyl)-pyridin-2-ylamine.¹H NMR (400 MHz, CDCl₃) δ 8.48 (m, 1H), 8.34 (m, 1H), 8.28 (br s, 1H), 7.90 (m, 1H), 7.10 (m, 2H), 6.85 (m, 1H), 4.46 (br s, 1H), 3.83 (m, 1H), 3.49-3.38 (m, 4H), 2.72 (m, 1H), 2.25 (m, 2H), 1.90-1.65 (m, 6H). ESI-MS m/z: 400.9 (M+H)⁺; t_{R} = 1.11min (Method A). The cis stereochemistry was assigned based on the chemical shift (3.83ppm) and splitting (m) of the methine proton at C-1 of the cyclohexane ring and on the chemical shift (2.72ppm) and splitting (m) of the methine proton at C-4 of the cyclohexane ring in the NMR spectrum.

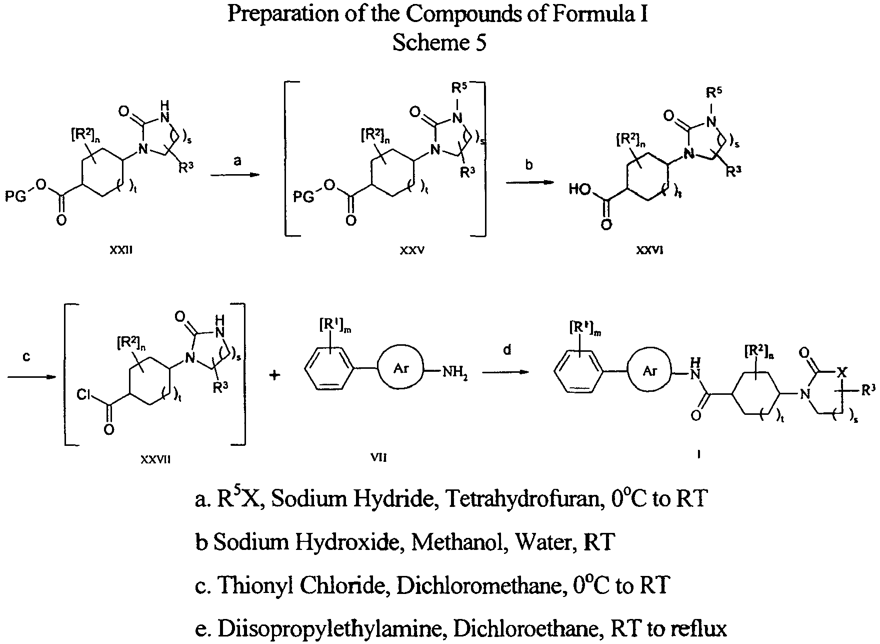

The compounds of Formula I wherein X is NR⁵ and s is 1 or 2 may be synthesized according to the procedures described in Scheme 5. A cyclic urea ester of Formula XXII is alkylated in the presence of a base and an alkyl halide to afford an N-alkyl urea ester of Formula XXVI which is directly hydrolyzed to an acid of Formula XXVII. Conversion to an acid chloride of Formula XXVIII followed by reaction with an amine of Formula VII affords the compounds of Formula I.

Representative intermediates were synthesized according to Scheme 5.

### Intermediate of Formula XXV, wherein R₃ is H, s is 2, and R⁵ is CH₃: cis-4-(3-Methyl-2-oxo-tetrahydro-pyrimidin-1-yl)-cyclohexanecarboxylic acid and trans-4-(3-Methyl-2-oxo-tetrahydro-pyrimidin-1-yl)-cyclohexanecarboxylic acid

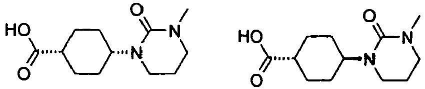

To a solution of 4-(2-oxo-tetrahydro-pyrimidin-1-yl)-cyclohexanecarboxylic acid ethyl ester (1.00g, 3.93mmol) in tetrahydrofuran (60mL) was added sodium hydride (0.190g of a 60% oil dispersion, 7.92mmol). After it was stirred at rt for 1h, iodomethane (0.600mL, 9.64mmol) was added and the resulting mixture was stirred at room temperature overnight. A solution of sodium hydroxide (0.50g, 12.5mmol) in water (6mL) was added and the mixture was stirred at room temperature overnight. The volatiles were removed in vacuo. The residue was dissolved in 6mL water and the pH was adjusted to ∼4 by addition of 12N HCL The mixture was transferred to a separatory funnel and extracted with dichloromethane (5x8 mL). The combined organic phases were dried over MgSO₄ and concentrated in vacuo to afford the crude product which was purified by mass-directed HPLC (Purified on a reversed phase liquid chromatography/mass spectrometry (RP-HPLC/MS) purification system. Gradient: acetonitrile in water, 9-95% in 2.8 minutes with a cycle time of 5 min. A shallow gradient between 10-25% of acetontrile was used between 0.5-2.5 min to separate cis and tans isomers. Flow rate: 100 mL/min. Mobile phase additive: 17 mM of acetic acid. Column: Inertsil C18, 30 x 50 mm, 5 um particle size.) to give cis product (0.28g) and trans product (0.24g). cis product, ¹H NMR (400 MHz, CDCl₃): δ 10.0 (br s, 1H), 4.37 (m, 1H), 3.22 (m, 2H), 3.15 (m, 2H), 2.94 (s, 3H), 2.67 (m, 1H), 224 (m, 2H), 1.90 (m, 2H), 1.73-1.53 (m, 6H). ESI-MS m/z: 241.0 (M+H)⁺; t_{R} = 1.19min (Method B). The cis stereochemistry was assigned based on the chemical shift (4.37ppm) and splitting (m) of the methine proton at C-1 of the cyclohexane ring and on the chemical shift (2.67ppm) and splitting (m) of the methine proton at C-4 of the cyclohexane ring in the NMR spectrum.
trans product, ¹H NMR (400 MHz, CDCl₃): δ 10.75 (br s, 1H), 4.30 (m, 1H), 3.21 (m, 2H), 3.15 (m, 2H), 2.93 (s, 3H), 2.18 (m, 1H), 2.07 (m, 2H), 1.91 (m, 2H), 1.74 (m, 2H), 1.60 (m, 2H), 1.41 (m, 2H). ESI-MS m/z: 241.0 (M+H)⁺; t_{R} = 1.35min (Method B). The trans stereochemistry was assigned based on the chemical shift (4.30ppm) and splitting (m) of the methine proton at C-1 of the cyclohexane ring in the NMR spectrum.

### Compounds of the Invention

The compounds of Examples 5a-5c were prepared according to the procedures in Scheme 5.

### Example 5a cis-4-(3-Methyl-2-oxo-tetrahydro-pyrimidin-1-yl)-cyclohexanecarboxylic acid [5-(3,5-difluoro-phenyl)-pyridin-2-yl]-amide

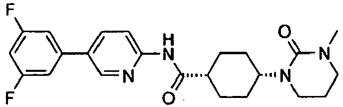

To a solution of cis-4-(3-methyl-2-oxo-tetrahydro-pyrimidin-1-yl)-cyclohexanecarboxylic acid (150mg, 0.624mmol) in methylene chloride (10mL) was added thionyl chloride (0.10mL, 1.37mmol) at rt. The mixture was stirred at rt for 3h. The volatiles were removed in vacuo. The residue was dissolved in 1,2-dichloroethane (20mL). 5-(3,5-Difluoro-phenyl)-pyridin-2-ylamine (130mg, 0.63mmol) and N,N-diisopropylethylamine (0.220mL, 1.26mmol) were added The reaction mixture was refluxed for 18h. After cooling to rt, the reaction mixture was transferred to a separatory funnel and washed with saturated NaHCO₃ followed by brine. The combined aqueous phases were extracted with methylene chloride. The combined organic phases were dried over MgSO₄ and concentrated in vacuo. The product was purified by MPLC using a solvent gradient from 30% ethyl acetate / 70% hexanes to 100% ethyl acetate (55 mg; Yield = 21%). ¹H NMR (400 MHz, CDCl₃): δ 8.48 (d, 1H), 8.35 (d, 1H), 8.25 (br s, 1H), 7.89 (dd, I H), 7.09 (m, 2H), 6.84 (m, 1H), 4.36 (m, 1H), 3.20 (m, 4H), 2.95 (s, 3H), 2.70 (m, 1H), 2.24 (m, 2H), 1.89 (m, 2H), 1.81 (m, 4H), 1.62 (m, 2H). ESI-MS m/z: 428.9 (M+H)⁺; t_{R} = 1.26min (Method A). The cis stereochemistry was assigned based on the chemical shift (4.36ppm) and splitting (m) of the methine proton at C-1 of the cyclohexane ring and on the chemical shift (2.70ppm) and splitting (m) of the methine proton at C-4 of the cyclohexane ring in the NMR spectrum.

Likewise, the following compounds were prepared analogously to that of Example 5a:

### Example 5b cis4-(3-Methyl-2-oxo-imidazolidin-1-yl)-cyclohexanecarboxylic acid [5-(3,5-difluoro-phenyl)-pyridin-2-yl]-amide

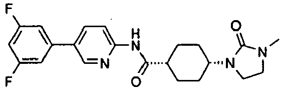

Prepared from cis-4-(3-methyl-2-oxo-imidazolidin-1-yl)-cyclohexanecarboxylic acid and 5-(3,5-difluoro-phenyl)-pyridin-2-ylamine. ¹H NMR (400 MHz, CDCl₃) δ 8.46 (m, 1H), 8.45 (br s, 1H), 8.31 (m, 1H), 7.89 (m, 1H), 7.08 (m, 2H), 6.83 (m, 1H), 3.80 (m, 1H), 3.33-3.22 (m, 4H), 2.78 (s, 3H), 2.70 (m, 1H), 2.22 (m, 2H), 1.90-1.68 (m, 6H). ESI-MS m/z: 414.9 (M+H)⁺; t_{R}= 1.25min (Method A). The cis stereochemistry was assigned based on the chemical shift (3.80ppm) and splitting (m) of the methine proton at C-1 of the cyclohexane ring and on the chemical shift (2.70ppm) and splitting (m) of the methine proton at C-4 of the cyclohexane ring in the NMR spectrum.

### Example 5c trans-4-(3-Methyl-2-oxo-imidazolidin-1-yl)-cyclohexanecarboxylic acid [5-(3,5-difluoro-phenyl)-pyridin-2-yl]-amide

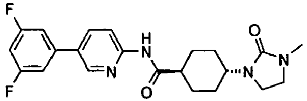

Prepared from trans-4-(3-methyl-2-oxo-imidazolidin-1-yl)-cyclohexanecarboxylic acid and 5-(3,5-difluoro-phenyl)-pyridin-2-ylamine. ¹H NMR (400 MHz, DMSO-d6) δ 10.62 (br s, 1H), 8.75 (m, 1H), 8.19 (m, 2H), 7.54 (m, 2H), 7.25 (m, 1H), 3.49 (m, 1H), 3.22 (m, 4H), 2.63 (s, 3H), 2.51 (m, 1H), 1.92 (m, 2H), 1.66 (m, 2H), 1.49 (m, 4H). ESI-MS m/z: 414.9 (M+H)⁺; t_{R} = 1.20min (Method A). The trans stereochemistry was assigned based on the chemical shift (3.49ppm) and splitting (m) of the methine proton at C-1 of the cyclohexane ring and on the chemical shift (2.51ppm) and splitting (m) of the methine proton at C-4 of the cyclohexane ring in the NMR spectrum.

### Example 5d cis-4-(3-Methyl-2-oxo-imidazolidin-1-yl)-cyclohexanecarboxylic acid [5-(3,5-difluoro-phenyl)-pyrimidin-2-yl]-amide

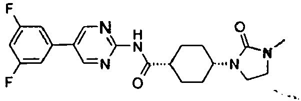

Prepared from cis-4-(3-methyl-2-oxo-imidazolidin-1-yl)-cyclohexanecarboxylic acid and 5-(3,5-difluoro-phenyl)-pyrimidin-2-ylamine. ESI-MS m/z: 416.0 (M+H)⁺; t_{R} = 0.91min (Method A).

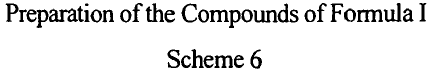

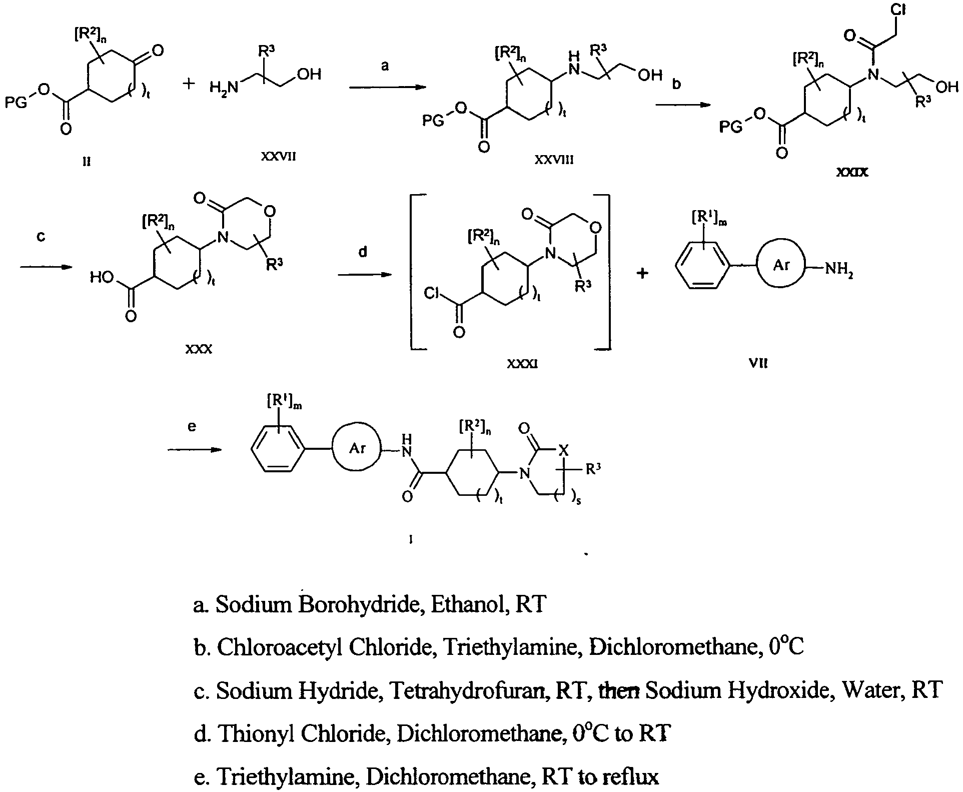

The compounds of Formula I, wherein X is CH₂O and s is I, may be synthesized according to the procedures described in Scheme 6. The starting materials of Formulas II and XXVII are commercially available or may be synthesized by procedures known in the prior art. In summary, an amino ester of Formula II is combined with an amino alcohol of Formula XXVII in the presence of a reducing agent to afford an amino alcohol of Formula XXVIII which is further treated with chloroacetyl chloride to form a chloroamide of Formula XXIX. Base treatment effects cyclization to a morpholinone and hydrolysis of the ester to afford an acid of Formula XXX. Conversion to an acid chloride of Formula XXXI followed by reaction with an amine of Formula VII affords the compounds of Formula I.

Representative intermediates were synthesized according to Scheme 6.

### Intermediate of Formula XXVIII, wherein R₃ is H and s is 1: 4-(2-Hydroxy-ethylamino)-cyclohexanecarboxylic acid ethyl ester

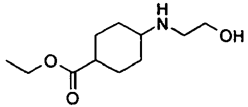

To a solution of 4-oxo-cyclohexanecarboxylic acid ethyl ester (3.85g, 22.6mmol) in ethanol (50mL) was added ethanolamine (1.36mL, 22.6mmol). The mixture was stirred for 1h at rt. Sodium tetrahydroborate (1.11g, 29.4mmol) was added and the resulting mixture was stirred at rt for 1h. The reaction was quenched by the addition of water (1.1mL). Methylene chloride (60 m) was added and the white solid was removed by filtration. The filtrate was concentrated in vacuo to afford the crude product as a mixture of cis and trans isomers which was used in the following reaction without further purification. ¹H NMR (400 MHz, CDCl₃) δ 4.12 (m, 2H), 3.63 (m, 2H), 2.87-2.74 (m, 2H), 2.66-1.00 (m, 15H). ESI-MS m/z: 216.1 (M+H)⁺; t_{R}= 0.23min (Method A).

### Intermediate of Formula XXIX, wherein R₃ is H and s is 1: 4-[(2-Chloro-acetyl)-(2-hydroxyethyl)-amino]-cyclohexanecarboxylic acid ethyl ester

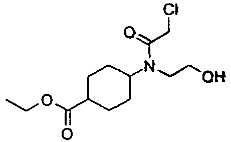

4-(2-Hydroxy-ethylamino)-cyclohexanecarboxylic acid ethyl ester (2.15g, 9.99mmol) and triethylamine (2.78mL, 20.0mmol) were dissolved in methylene chloride (20mL) at 0 °C. Chloroacetyl chloride (0.800mL, 10.0mmol) in 20mL methylene chloride was added dropwise. The mixture was stirred for 3h. The mixture was transferred to a separatory funnel and washed with saturated sodium bicarbonate and followed by brine. The organic phase was dried over MgSO₄ and concentrated in vacuo. The residue was purified by MPLC using a solvent gradient from 50% ethyl acetate / 50% hexanes to 100% ethyl acetate to give the title compound as a mixture of cis and trans isomers (1.89 g; Yield = 64.9%). ¹H NMR (400 MHz, CDCl₃) δ 4.20-4.07 (m, 4H), 3.73 (m, 2H), 3.61 (m, 1H), 3.47 (m, 2H), 2.70-1.24 (m, 13H). ESI-MS m/z: 292.0 (M-+H)⁺; t_{R}= 0.73min, ESI-MS m/z: 292.0 (M+H)⁺; t_{R}= 0.76min (Method A).

### Intermediate of Formula XXX, wherein R₃ is H and s is 1: 4-(3-Oxo-morpholin-4-yl)-cyclohexanecarboxylic acid

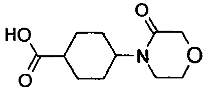

To a solution of 4-[(2-chloro-acetyl)-(2-hydroxy-ethyl)-amino]-cyclohexanecarboxylic acid ethyl ester (511mg, 1.75mmol) in tetrahydrofuran (15mL) was added sodium hydride (84.0mg of a 60% dispersion in mineral oil, 3.50mmol). The mixture was stirred at rt for 20 min. A sodium hydroxide solution (2.0M, 1.0mL) and methanol (20mL) were added. The resulting mixture was stirred at room temperature overnight. The volatiles were removed in vacuo. The residue was dissolved in water (5mL) and the pH adjusted to ∼4 with 3N HCl. The solution was transferred to a separatory funnel and extracted with methylene chloride. The combined organic phases were dried over MgSO₄ and concentrated in vacuo to give the title compound as a mixture of cis and trans isomers. (0.312 g; Yield = 78.4%). ¹H NMR (400 MHz, CDCl₃) δ 10.20 (br s, 1H), 4.45 (m, 1H), 4.20 (s, 2H), 3.86 (m, 2H), 3.27 (m, 2H), 2.75-1.40 (m, 9H). ESI-MS m/z: 228.0 (M+H)⁺; t_{R}=0.95min. ESI-MS m/z: 228.0 (M+H)⁺; t_{R}= 1.03min (Method B).

### Compounds of the Invention

The compounds of Examples 6a-6h were prepared according to the procedures in Scheme 6.

### Examples 6a and 6b cis-4-(3-Oxo-morpholin-4-yl)-cyclohexanecarboxylic acid [5-(3,5-difluorophenyl)-pyridin-2-yl]-amide and trans-4-(3-Oxo-morpholin-4-yl)-cyclohexanecarboxylic acid [5-(3,5-difluoro-phenyl)-pyridin-2-yl]-amide

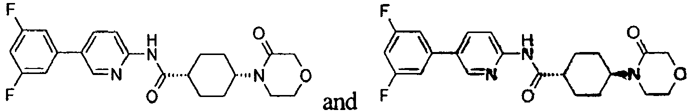

To a solution of 4-(3-oxo-morpholin-4-yl)-cyclohexanecarboxylic acid (0.310g, 1.36mmol) in methylene chloride (10mL) at 0 °C was added thionyl chloride (0.200mL, 2.74mmol). The mixture was stirred at room temperature for 3h. The volatiles were removed in vacuo to afford the intermediate acid chloride. This was dissolved in methylene chloride (10mL) and added to a solution of triethylamine (1.00mL, 7.17mmol) and 5-(3,5-difluoro-phenyl)-pyridin-2-ylamine (0.281g, 1.36mmol) in 10mL methylene chloride at 0 °C . The mixture was refluxed for 18h. After cooling to rt, the reaction mixture was transferred to a separatory funnel and washed with brine. The aqueous phase was extracted with methylene chloride. The combined organic phases were dried over MgSO₄, filtered, and concentrated in vacuo. The residue was purified by HPLC using a solvent gradient from 30% ethyl acetate / 70% hexanes to 100% ethyl acetate to give the desired products.

CIS product 40 mg, ¹H NMR (400 MHz, CDCl₃): δ 8.40 (dd, 1H), 8.23 (dd, 1H), 8.02 (br s, 1H), 7.81 (dd, 1H), 7.01 (m, 2H), 6.76 (m, 1H), 4.50 (m, 1 H), 4.12 (s, 2H), 3.78 (m, 2H), 3.24 (m, 2H), 2.64(m, 1H), 2.20 (m, 2H), 1.79 (m, 2H), 1.59 (m, 4H). ESI-MS m/z: 415.9 (M+H)⁺; t_{R} = 1.15min (Method A). The cis stereochemistry was assigned based on the chemical shift (4.50ppm) and splitting (m) of the methine proton at C-1 of the cyclohexane ring and on the chemical shift (2.64ppm) and splitting (m) of the methine proton at C-4 of the cyclohexane ring in the NMR spectrum.

TRANS product 96mg, ¹H NMR (400 MHz, DMSO-d6): δ 10.65 (br s, 1H), 8.75 (s, 1H), 8.19 (s, 2H), 7.55 (d, 2H), 7.25 (m, 1H), 4.22 (m, 1H), 4.03 (s, 2H), 3.81 (m, 2H), 3.27 (m, 2H), 2.50 (m, 1H), 1.95 (m, 2H), 1.68-1.47 (m, 6H). ESI-MS m/z: 415.9 (M+H)⁺; t_{R} = 1.11min (Method A). The trans stereochemistry was assigned based on the chemical shift (4.22ppm) and splitting (m) of the methine proton at C-1 of the cyclohexane ring and on the chemical shift (2.50ppm) and splitting (m) of the methine proton at C-4 of the cyclohexane ring in the NMR spectrum.

Likewise, the following compounds were prepared analogously to that of Examples 6a and 6b:

### Example 6c trans-4-(3-Oxo-morpholin-4-yl)-cyclohexanecarboxylic acid [1-(3,5-difluorophenyl)-1H-pyrazol-4-yl]-amide

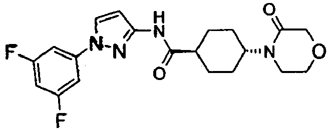

Prepared from trans-4-(3-oxo-morpholin-4-yl)-cyclohexanecarboxylic acid and 1-(3,5-difluorophenyl)-1H-pyrazol-3-ylamine. ¹H NMR (400 MHz, CDCl₃): δ 7.92 (br s, 1H), 7.81 (d, 1H), 7.17 (m, 2H), 7.02 (d, 1H), 6.71 (m, 1H), 4.51 (m, 1H), 4.21 (s, 2H), 3.91 (m, 2H), 3.32 (m, 2H), 2.30-2.11 (m, 3H), 1.94-1.70 (m, 4H), 1.66-1.50 (m, 2H). ESI-MS m/z: 404.9 (M+H)⁺; t_{R} = 0.98min (Method A).

### Example 6d cis-4-(3-Oxo-morpholin-4-yl)-cyclohexanecarboxylic acid [1-(3,5-difluoro-phenyl)-1 H-pyrazol-4-yl]-amide

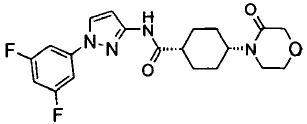

Prepared from cis-4-(3-oxo-morpholin-4-yl)-cyclohexanecarboxylic acid and 1-(3,5-difluorophenyl)-1H-pyrazol-3-ylamine. ¹H NMR (400 MHz, CDCl₃): δ 7.97 (br s, 1H), 7.82 (d, 1H), 7.18 (m, 2H), 7.00 (d, 1H), 6.72 (m, 1H), 4.58 (m, 1H), 4.21 (s, 2H), 3.78 (m, 2H), 3.32 (m, 2H), 2.70 (m, 1H), 2.26 (m, 2H), 1.95-1.75 (m, 4H), 1.70-1.60 (m, 2H). ESI-MS m/z: 404.9 (M+H)⁺; t_{R} = 1.02min (Method A).

### Example 6e trans-4-(3-Oxo-morpholin-4-yl)-cyclohexanecarboxylic acid [1-(2-fluoro-phenyl)-1H-pyrazol-4-yl]-amide

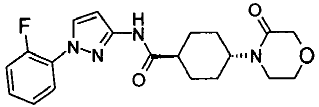

Prepared from trans-4-(3-oxo-morpholin-4-yl)-cyclohexanecarboxylic acid and 1-(2-fluorophenyl)-1H-pyrazol-3-ylamine. ¹H NMR (400 MHz, CDCl₃): δ 8.21 (br s, 1H), 7.93 (t, 1H), 7.77 (m, 1H), 7.25 (m, 3H), 7.00 (d, 1H), 4.48 (m, 1H), 4.21 (s, 2H), 3.90 (m, 2H), 3.30 (m, 2H), 2.13 (m, 3H), 1.89-1.68 (m, 4H), 1.59-1.42 (m, 2H). ESI-MS m/z: 386.9 (M+H)⁺; t_{R} = 0.85min (Method A).

### Example 6f cis-4-(3-Oxo-morpholin-4-yl)-cyclohexanecarboxylic acid [1-(2-fluoro-phenyl)-1H-pyrazol-4-yl]-amide

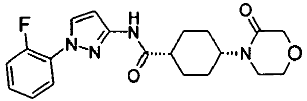

Prepared from cis-4-(3-oxo-morpholin-4-yl)-cyclohexanecarboxylic acid and 1-(2-fluoro-phenyl)-1H-pyrazol-3-ylamine. ¹H NMR (400 MHz, CDCl₃): δ 8.25 (br s, 1H), 7.93 (t, 1H), 7.75 (m, 1H), 7.26 (m, 3H), 7.00 (d, 1H), 4.60 (m, 1 H), 4.20 (s, 2H), 3.86 (m, 2H), 3.32 (m, 2H), 2.68 (m, 1H), 2.25 (m, 2H), 2.00-1.73 (m, 4H), 1.68-1.59 (m, 2H). ESI-MS m/z: 386.9 (M+H)⁺; t_{R} = 0.89min (Method A).

### Example 6g cis-4-(3-Oxo-morpholin-4-yl)-cyclohexanecarboxylic acid [5-(3,5-difluoro-phenyl)-pyrazin-2-yl]-amide

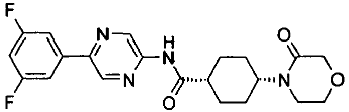

Prepared from cis-4-(3-oxo-morpholin-4-yl)-cyclohexanecarboxylic acid and 5-(3,5-difluorophenyl)-pyrazin-2-ylamine. ¹H NMR (400 MHz, CDCl₃): δ 9.60 (d, 1H), 8.65 (d, 1H), 7.97 (br s, 1H), 7.55(m, 2H), 6.90 (m, 1H), 4.60 (m, 1H), 4.21 (s, 2H), 3.88 (m, 2H), 3.33 (m, 2H), 2.77 (m, 1H), 2.28 (m, 2H), 2.01-1.81 (m, 4H), 1.73-1.64 (m, 2H). ESI-MS m/z: 416.9 (M+H)⁺; t_{R} = 1.16min (Method A).

### Example 6h cis-4-(3-Oxo-morpholin-4-yl)-cyclohexanecarboxylic acid [5-(3,5-difluoro-phenyl)-pyrimidin-2-yl]-amide

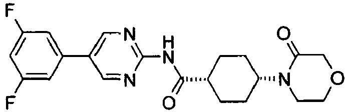

Prepared from cis-4-(3-oxo-morpholin-4-yl)-cyclohexanecarboxylic acid and 5-(3,5-difluorophenyl)-pyrimidin-2-ylamine. ¹H NMR (400 MHz, CDCl₃): δ 8.81 (s, 2H), 8.26 (br s, 1H), 7.07(m, 2H), 6.91 (m, 1H), 4.59 (m, 1H), 4.21 (s, 2H), 3.86 (m, 2H), 3.34 (m, 2H), 2.98 (m, 1H), 2.25 (m, 2H), 2.02 (m, 2H), 1.84 (m, 2H), 1.63 (m, 2H). ESI-MS m/z: 416.9 (M+H)⁺; t_{R} = 0.86min (Method A).

### Example 7 In-Vitro Pharmacological Evaluation at Cloned Neuropeptide Y5 Receptor.

The pharmacological properties of the compounds of the present invention were evaluated at the cloned human NPY Y5 receptor using the protocols disclosed in U.S. Patent No. 6,124,331, the contents of which are hereby incorporated by reference. The procedures for cell culture, transient transfection and membrane harvest, which are well known in the art, are described therein

### Radioligand Binding to Membrane Suspensions

Briefly, membrane suspensions from transfected cells (typically expressed in LM(tk-) cells) and ¹²⁵I-PYY radioligand (PerkinElmer, Waltham, MA) were diluted in binding buffer supplemented with 0.1 % bovine serum albumin to yield an optimal membrane protein concentration so that ¹²⁵I-PYY bound by membranes in the assay was less than 10 % of ¹²⁵I-PYY delivered to the sample (100,000 dpm/ sample = 0.08 nM for competition binding assays). Test compounds were diluted to desired concentrations with supplemented binding buffer in the presence of 30% DMSO. The binding assay was performed in 96-well polypropylene microtiter plates by mixing ¹²⁵I-PYY, test compound (25 µL), and finally, membrane suspensions (200 µL). Final DMSO = 3%. Samples were incubated in at room temperature for about 120 min. Incubations were terminated by filtration over Whatman GF/C filters (pre-coated with 1% polyethyleneimine and air-dried before use), followed by washing with ice-cold binding buffer. Filter-trapped membranes were impregnated with MeltiLex solid scintillant (Wallac, Turku, Finland) and counted for ¹²⁵I-PYY in a Wallac MicroBeta Trilux. Non-specific binding was defined by 1000 nM porcine NPY. Specific binding was typically 80 %; most non-specific binding was associated with the filter. Binding data were analyzed using nonlinear regression and statistical techniques available in the GraphPAD Prism package (San Diego, Calif.).

The binding affinities for the compounds in the present invention, exemplified above, at the human NPY Y5 receptor were determined to be about 10 µM or less. The binding affinities for most of the compounds were determined to be about 1.0 µM or less. The binding affinities for several compounds were determined to be about 100 nM or less. A few compounds bind to the receptor with binding affinity less than about 10nM.

**Table I: binding affinity of selected compounds**

| Example No. | 1a | 2a | 3a | 4a | 5a | 5b | 6a |
|---|---|---|---|---|---|---|---|
| Ki (nM) | 720 | 8 | 6 | 9 | 12 | 3.8 | 8 |

### Functional Assay: NPY Y5-Dependent Inhibition of Forskolin-stimulated cAMP Accumulation

Stably transfected cells were seeded into 96-well microtiter plates and cultured until confluent. To reduce the potential for receptor desensitization, the serum component of the media was reduced to 1.5% for 4 to 16 hours before the assay. Cells were washed in Hank's buffered saline, or HBS (150 mM NaCl, 20 mM HEPES, 1 mM CaCl₂, 5 mM KCI, 1 mM MgCl₂, and 10 mM glucose) supplemented with 0.1% bovine serum albumin plus 100 uM IBMX. Test compounds were diluted to desired concentrations with assay buffer in the presence of 10% DMSO, then transferred to the cell plate and allowed to incubate for 20 min at 37 °C in 5% CO₂ (final DMSO = 1%). Cells were then stimulated with NPY (up to 10 uM) for a period of 5 min, followed by forskolin (10 uM) for another 5 min. The assay was then terminated and intracellular cAMP was quantified by with highthroughput time-resolved flourometry (HTRF kit from CisBio, Bedford, MA). The effect of the test compound on agonist (NPY) activity were analyzed using nonlinear regression and statistical techniques available in the GraphPAD Prism package (San Diego, CA). The compounds of Example 2a and 5b were determined to function as an antagonist at the NPY Y5 receptor.

### Example 8 In-Vivo Assays

The in-vivo effects of the compounds of the present invention can be evaluated by using the following in-vivo behavioral animal models. The behavioral models described below are not intended to be the only models used to determine the efficacy of a compound of the invention to treat the corresponding disorder.

Agonist-stimulated feeding assay. Sprague-Dawley rats (250-275 g) are implanted with guide cannulae into the lateral cerebral ventricle at Charles River Laboratories (Kingston, NY) and shipped to the animal facility 2-3 days later. After a I week acclimation period, successful cannulae placement was confirmed by robust drinking in response to an i.c.v. infusion of angiotensin II (100 µg). At least 5 days prior to initiation of feeding studies, rats are acclimated to cages with wire grid floors, suspended by a stainless steel support above a waste tray. Food was removed on the morning of testing. To study the blockade of Y₅ receptor-induced feeding by the compound, animals were dosed orally with vehicle alone (20% cyclodextrin in distilled H₂O) or the compound , followed 1 h later by i.c.v. infusion (5 µl over 1 min) of the Y₅ receptor-selective peptide agonist cPP (0.6 nmol in normal saline). The 0.6 nmol dose of cPP was selected because it is just below the ED₅₀ dose (0.75 nmol) determined from preliminary experiments (data not shown) and provides a robust feeding signal. Animals are returned to the feeding cages and a pre-weighed amount of food was made available for 1 h. Net food intake = pre-weighed amount - (final amount + spilled amount). To test for nonspecific inhibition of the feeding response, we assess the effect of the compound (30 mg/kg) on feeding induced by 2 nmol NPY, a dose that evoked a feeding response equivalent to that of 0.6 nmol cPP.

Rat Forced-swim Test: The procedure which may be used here is similar to that previously described (Luki, et al. Psychopharmacology 2001, 155, 315-322) with the following modifications. Male Sprague-Dawley rats may be used. Swim sessions are conducted for about 5 min, by placing rats in a plexiglass cylinder (about 46 cm tall x 20 cm in diameter) filled about 30 cm deep with water at about 23°C. A compound of the invention or vehicle (about 0.01% lactic acid, about pH 6) is administered orally as a 1ml/kg solution. Test sessions are videotaped and recorded for later scoring by a single rater, who is blinded to the treatment condition. Immobility is scored as the time a rat remained floating in the water making only movements necessary to keep its head above the water. Swimming is scored as the time a rat made active swimming motions, more than necessary to maintain its head above water.

Rat Social-interaction Test: The procedure is performed for about 15 min as previously described (File and Hyde Br. J. Pharmacol. 1987, 62, 19-24) under low-light conditions using pairs of unfamiliar male Sprague-Dawley rats previously housed singly and exposed to the test arena for about 15 min on the previous day. A compound of the invention, chlordiazepoxide or vehicle is injected i.p. as a ~ 1.0 ml/kg solution. All test sessions are videotaped and recorded for later scoring. Active social interaction, defined as sniffing, grooming, biting, boxing and crawling over and under, as well as locomotor activity (defined as squares crossed), is scored by a single rater, who is blinded to the treatment of each pair.

Chronic mild stress: The chronic mild stress (CMS) test is performed using male Wistar rats as described previously (Papp et al., 2002). Rats are first trained to consume a 1% sucrose solution in a series of baseline tests during which the sucrose solution was presented in the home cage for 1 h following 14 h food and water deprivation. On the basis of their final sucrose intake scores, animals are divided into two matched groups, one subjected continuously to chronic mild stress for a period of 8-9 consecutive weeks and the other housed separately as a non-stressed control group. Both groups are subjected to a sucrose consumption test once weekly, around 10:00 a.m. and under similar conditions as in the training period. Following 2-3 weeks of stress, sucrose intake scores are used to further divide both stressed and control animals into matched groups (n = 8 per group). For the next 5 weeks both stressed and control animals receive twice daily intraperitoneal injections of vehicle (0.25% hydroxypropyl β methylcellulose, 1 ml/kg), a compound of the invention or citalopram at approx. 10:00 and 17:00, except that the 17:00 i.p. injection was omitted on days preceding the sucrose test. After 5 weeks the i.p. injections were terminated in both groups. Twenty-four h after the last i.p. injection, all animals were sacrificed and brains removed.

Novel Object Recognition: Novel Object Recognition is a hippocampus-dependent, non-spatial, visual memory task that takes advantage of rodent's innate drive to explore novelty in its environment. Vehicle and/or test compound were administered at a dosing volume according to experimental design (i.p., p.o., or s.c.) at an interval ranging from 30 minutes to 24 hours prior to training or testing. Step 1: Animals were transported in their home cages from the vivarium to the dimly lit (5-6 lux) training room and are acclimated for approximately 45 minutes. Step 2: Animals were individually placed in the training/testing chamber for 3-15 minutes of habituation. The animals were then returned to their home cage. They remain in the testing room for approximately 15 minutes after the last animal is habituated before being returned to their colony room. Step 3: The day after initial habituation, the animals were returned to the testing room and acclimated as described in Step 1. The training procedure begins by placing the animal, nose facing the wall, into the empty chamber at a position in the center of a long wall. After 1 minute of re-habituation, the animal was removed from the chamber and placed in a holding cage for a period of 10 seconds. During this delay, two identical objects were placed 8 cm from the sides of the two short walls of the chamber; object pairs are randomly assigned within and between conditions. The animal was then returned to the chamber for a 3-15 minute period of exploration. After the training session, the animal is returned to its home cage. The animals remain in the testing room for approximately 15 minutes after the last animal was trained before being returned to their colony room. Step 4. Following a delay interval (1 hour to several weeks, but usually 24 hours), the animals were again returned to the testing room and acclimated as described in Step 1. After this, individual animals are re-habituated to the empty chamber for 1 minute as was described in Step 3. For the test, two new objects are placed in the chamber: one was identical to the objects used during training, while the other was novel. The position of the novel object was randomized for each animal. The animals were allowed to actively explore for 3-15 minutes. During the test period, the amount of time spent exploring either the novel or the familiar object was recorded using a hand held timer and video tape recorder or computer-based object exploration analysis program.

## Claims

1. A compound of Formula I: 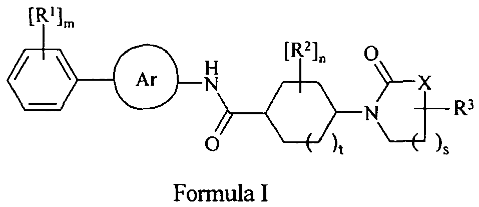 wherein R¹ is C₁-C₇ alkyl, C₁-C₇perfluoroalkyl, C₁-C₇ alkoxy or halogen;
wherein R² is C₁-C₇ alkyl;
wherein R³ is H, C₁-C₇ alkyl, C₁-C₇ alkoxy, -(CH₂)ᵤOH, -N(R⁴)C(O)C₁-C₇ alkyl or - N(R⁴)C(O)C₁-C₇ alkoxy;
wherein R⁴ is H or C₁-C₇ alkyl;
wherein X is -CH₂-, -0-, -CH₂O-, -NR⁵- or -CH₂NR⁵-;
wherein R⁵ is H, C₁-C₇ alkyl, phenyl or pyridyl, where the phenyl and pyridyl are optionally substituted with C₁-C₇ alkyl, C₁-C₇perfluoroalkyl, C₁-C₇ alkoxy or halogen;
wherein Ar is a divalent aromatic moiety selected from the group consisting of phenyl, pyridine, pyrimidine, pyridazine, pyrazine, imidazole, pyrazole, triazole, oxazole and isoxazole;
wherein each m and n is independently an integer from 0 to 5 inclusive;
wherein u is 0 or 1; and
wherein each t and s is independently an integer from 0 to 2 inclusive; of a pharmaceutically acceptable salt thereof.

2. The compound of claim 1, wherein Ar is phenyl.

3. The compound of claim 1, wherein Ar is pyridine or pyrimidine.

4. The compound of claim 1, wherein Ar is pyridazine, pyrazine or triazole.

5. The compound of claim 1, wherein Ar is imidazole, pyrazole, oxazole or isoxazole.

6. The compound of claim 1, wherein t is I and where the cyclohexane moiety is in the cis configuration.

7. The compound of claim 1, wherein t is 1 and where the cyclohexane moiety is in the trans configuration.

8. The compound of claim 1, wherein X is -CH₂-.

9. The compound of claim 1, wherein X is -O-.

10. The compound of claim 1, wherein X is -CH₂O-.

11. The compound of claim 1, wherein X is -NR⁵- and wherein R⁵ is H or C₁-C₄ alkyl.

12. The compound of claim 1, wherein X is -CH₂NR⁵-; and wherein R⁵ is H or C₁-C₄ alkyl

13. The compound of claim 1, wherein R¹ is C₁-C₄ alkoxy or C₁-C₄ perfluoroalkyl; and wherein m is 0, 1 or 2.

14. The compound of claim 1, wherein R¹ is C₁-C₄ alkyl, F or Cl; and wherein m is 0, 1 or 2.

15. The compound of claim 1, wherein n is 0.

16. The compound of claim 1, wherein s is 0 or 1.

17. The compound of claim 1, wherein R³ is H.

18. The compound of claim 1, wherein R³ is C₁-C₄ alkyl, C₁-C₄ alkoxy or -(CH₂)ᵤOH; and u is 0.

19. The compound of claim 1, wherein R³ is C₁-C₄ alkyl, C₁-C₄ alkoxy or -(CH₂)ᵤOH; and u is 1.

20. The compound of claim 1, wherein R³ is N(R⁴)C(O)C₁-C₄ alkyl, or -N(R⁴)C(O)C₁-C₄ alkoxy; and wherein R⁴ is H or C₁-C₄ alkyl.

21. The compound of claim 1, wherein the compound is selected from the group consisting of trans-4-(2-Oxo-pyrrolidin-1-yl)-cyclohexanecarboxylic acid [5-(3, 5-difluoro-phenyl)-pyridin-2-yl]-amide; cis-4-(2-Oxo-pyrrolidin-1-yl)-cyclohexanecarboxylic acid [5-(3,5-difluoro-phenyl)-pyridin-2-yl]-amide; cis4-(2-Oxo-pyrrolidin-1-yl)-cyclohexanecarboxylic acid [5-(3,5-dichloro-phenyl)-pyridin-2-yl]-amide; cis-4-(2-Oxopyrrolidin-1-yl)-cyclohexanecarboxylic acid [5-(3,5-difluoro-phenyl)-pyrazin-2-yl]-amide; 4-(2-Oxo-azetidin-1-yl)-cyclohexanecarboxylic acid [5-(3,5-difluoro-phenyl)-pyridin-2-yl]-amide; cis-4-(2-Oxo-piperidin-1-yl)-cyclohexanecarboxylic acid [5-(3,5-difluorophenyl)-pyridin-2-yl]-amide; cis-4-(2-Oxo-pyrrolidin-1-yl)-cyclohexanecarboxylic acid [5-(3-fluoro-5-methyl-phenyl)-pyridin-2-yl]-amide; cis-4-(2-Oxo-pyrrolidin-1-yl)-cyclohexanecarboxylic acid [5-(3-chloro-5-methyl-phenyl)-pyridin-2-yl]-amide; cis-4-(2-Oxo-oxazolidin-3-yl)-cyclohexanecarboxylic acid [5-(3,5-difluoro-phenyl)-pyridin-2-yl]-amide; trans-4-(2-Oxo-oxazolidin-3-yl)-cyclohexanecarboxylic acid [5-(3,5-difluorophenyl)-pyridin-2-yl]-amide; cis-4-(2-Oxo-oxazolidin-3-yl)-cyclohexanecarboxylic acid [5-(3,5-difluoro-phenyl)-pyrazin-2-yl]-amide; cis- 4-(2-Oxo-oxazolidin-3-yl)-cyclohexanecarboxylic acid [1-(3,5-difluoro-phenyl)-1H-pyrazol-3-yl]-amide; trans-4-(2-Oxo-[1,3]oxazinan-3-yl)-cyclohexanecarboxylic acid [1-(3,5-difluoro-phenyl)-1H-pyrazol-4-yl]-amide; cis-4-(2-Oxo-[1,3]oxazinan-3-yl)-cyclohexanecarboxylic acid [1-(3,5-difluoro-phenyl)-1H-pyrazol-4-yl]-amide; trans-4-(2-Oxo-[1,3]oxazinan-3-yl)-cyclohexanecarboxylic acid [1-(2-fluoro-phenyl)-1H-pyrazol-4-yl]-amide; cis-4-(2-Oxo-[1,3]oxazinan-3-yl)-cyclohexanecarboxylic acid [1-(2-fluoro-phenyl)-1H-pyrazol-4-yl]-amide; cis-4-(2-Oxo-[1,3]oxazinan-3-yl)-cyclohexanecarboxylic acid [5-(3,5-difluorophenyl)-pyrazin-2-yl]-amide; cis-4-(2-Oxo-tetrahydro-pyrimidin-1-yl)-cyclohexanecarboxylic acid [5-(3,5-difluoro-phenyl)-pyridin-2-yl]-amide; trans-4-(2-Oxo-tetrahydro-pyrimidin-1-yl)-cyclohexanecarboxylic acid [5-(3,5-difluoro-phenyl)-pyridin-2-yl]-amide; cis-4-(2-Oxo-imidazolidin-1-yl)-cyclohexanecarboxylic acid [5-(3,5-difluoro-phenyl)-pyridin-2-yl]-amide; cis-4-(3-Methyl-2-oxo-tetrahydro-pyrimidin-1-yl)-cyclohexanecarboxylic acid [5-(3,5-difluoro-phenyl)-pyridin-2-yl]-amide; cis-4-(3-Methyl-2-oxo-imidazolidin-1-yl)-cyclohexanecarboxylic acid [5-(3,5-difluoro-phenyl)-pyridin-2-yl]-amide; trans-4-(3-Methyl-2-oxo-imidazolidin-1-yl)-cyclohexanecarboxylic acid [5-(3,5-difluoro-phenyl)-pyridin-2-yl]-amide; cis-4-(3-Oxo-morpholin-4-yl)-cyclohexanecarboxylic acid [5-(3,5-difluoro-phenyl)-pyridin-2-yl]-amide; trans-4-(3-Oxo-morpholin-4-yl)-cyclohexanecarboxylic acid [5-(3,5-difluoro-phenyl)-pyridin-2yl]-amide; trans-4-(3-Oxo-morpholin-4-yl)-cyclohexanecarboxylic acid [1-(3,5-difluorophenyl)-1H-pyrazol-4-yl]-amide; cis-4-(3-Oxo-morpholin-4-yl)-cyclohexanecarboxylic acid [1-(3,5-difluoro-phenyl)-1H-pyrazol-4-yl]-amide; trans-4-(3-Oxo-morpholin-4-yl)-cyclohexanecarbolic acid [1-(2-fluoro-phenyl)-1H-pyrazol-4-yl]-amide; cis-4-(3-Oxo-morpholin-4-yl)-cyclohexanecarboxylic acid [1-(2-fluoro-phenyl)-1H-pyrazol-4-yl]-amide; cis-4-(3-Oxo-morpholin-4-yl)-cyclohexanecarboxylic acid [5-(3,5-difluorophenyl)-pyrazin-2-yl]-amide; and cis-4-(3-Oxo-morpholin-4-yl)-cyclohexanecarboxylic acid [5-(3,5-difluoro-phenyl)-pyrimidin-2-yl]-amide; cis-4-(2-Oxo-pyrrolidin-1-yl)-cyclohexanecarboxylic acid [5-(3,5-difluoro-phenyl)-pyrimidin-2-yl]-amide; cis-4-(2-Oxo-oxazolidin-3-yl)-cyclohexanecarboxylic acid [5-(3,5-difluoro-phenyl)-pyrimidin-2-yl]-amide and cis-4-(3-Methyl-2-oxo-imidazolidin-1-yl)-cyclohexanecarboxylic acid [5-(3,5-difluoro-phenyl)-pyrimidin-2-yl]-amide.

22. A pharmaceutical composition comrising a therapeutically effective amount of the compound of anyone of claims 1-21 and a pharmaceutically acceptable carrier.

23. Use of a compound of anyone of claims 1-21 for the manufacture of a medicament useful for treating ADHD.

24. Use of a compound of anyone of claims 1-21 for the manufacture of a medicament useful for treating a cognitive disorder.

25. Use of a compound of anyone of claims 1-21 for the manufacture of a medicament useful for treating schizophrenia.

## Patentansprüche

1. Verbindung mit der Formel I: 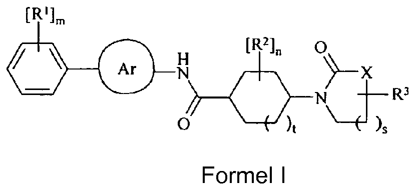 wobei R¹ C₁-C₇-Alkyl, C₁-C₇-Perfluoralkyl, C₁-C₇-Alkoxy oder Halogen ist;
wobei R² C₁-C₇-Alkyl ist;
wobei R³ H, C₁-C₇-Alkyl, C₁-C₇-Alkoxy, -(CH₂)ᵤOH, -N(R⁴)C(O)C₁-C₇-Alkyl oder -N(R⁴)C(O)C₁-C₇-Alkoxy ist;
wobei R⁴ H oder C₁-C₇-Alkyl ist;
wobei X -CH₂-, -O-, -CH₂O-, -NR⁵- oder -CH₂NR⁵- ist;
wobei R⁵ H, C₁-C₇-Alkyl, Phenyl oder Pyridyl ist, wobei das Phenyl und Pyridyl optional mit C₁-C₇-Alkyl, C₁-C₇-Perfluoralkyl, C₁-C₇-Alkoxy oder Halogen substituiert sind;
wobei Ar ein zweiwertiger aromatischer Anteil ist, der aus der Gruppe ausgewählt ist, die aus Phenyl, Pyridin, Pyrimidin, Pyridazin, Pyrazin, Imidazol, Pyrazol, Triazol, Oxazol und Isoxazol besteht;
wobei jedes m und n unabhängig eine Ganzzahl von 0 bis einschließlich 5 ist;
wobei u 0 oder 1 ist; und
wobei jedes t und s unabhängig eine Ganzzahl von 0 bis einschließlich 2 ist; oder ein pharmazeutisch akzeptables Salz davon.

2. Verbindung nach Anspruch 1, wobei Ar Phenyl ist.

3. Verbindung nach Anspruch 1, wobei Ar Pyridin oder Pyrimidin ist.

4. Verbindung nach Anspruch 1, wobei Ar Pyridazin, Pyrazin oder Triazol ist.

5. Verbindung nach Anspruch 1, wobei Ar Imidazol, Pyrazol, Oxazol oder Isoxazol ist.

6. Verbindung nach Anspruch 1, wobei t 1 ist und wobei der Cyclohexan-Anteil in der cis-Konfiguration ist.

7. Verbindung nach Anspruch 1, wobei t 1 ist und wobei der Cyclohexan-Anteil in der trans-Konfiguration ist.

8. Verbindung nach Anspruch 1, wobei X -CH₂- ist.

9. Verbindung nach Anspruch 1, wobei X -O- ist.

10. Verbindung nach Anspruch 1, wobei X -CH₂O- ist.

11. Verbindung nach Anspruch 1, wobei X -NR⁵- ist und wobei R⁵ H oder C₁-C₄-Alkyl ist.

12. Verbindung nach Anspruch 1, wobei X -CH₂NR⁵- ist; und wobei R⁵ H oder C₁-C₄-Alkyl ist.

13. Verbindung nach Anspruch 1, wobei R¹ C₁-C₄-Alkoxy oder C₁-C₄-Perfluoralkyl ist; und wobei m 0, 1 oder 2 ist.

14. Verbindung nach Anspruch 1, wobei R¹ C₁-C₄-Alkyl, F oder Cl ist; und wobei m 0, 1 oder 2 ist.

15. Verbindung nach Anspruch 1, wobei n 0 ist.

16. Verbindung nach Anspruch 1, wobei s 0 oder 1 ist.

17. Verbindung nach Anspruch 1, wobei R³ H ist.

18. Verbindung nach Anspruch 1, wobei R³ C₁-C₄-Alkyl, C₁-C₄-Alkoxy oder -(CH₂)ᵤOH ist; und u 0 ist.

19. Verbindung nach Anspruch 1, wobei R³ C₁-C₄-Alkyl, C₁-C₄-Alkoxy oder -(CH₂)ᵤOH ist; und u 1 ist.

20. Verbindung nach Anspruch 1, wobei R³ -N(R⁴)C(O)C₁-C₄-Alkyl oder -N(R⁴)C(O)C₁-C₄-Alkoxy ist; und wobei R⁴ H oder C₁-C₄-Alkyl ist.

21. Verbindung nach Anspruch 1, wobei die Verbindung aus der Gruppe ausgewählt ist, bestehend aus trans-4-(2-Oxopyrrolidin-1-yl)cyclohexancarbonsäure[5-(3,5-difluorphenyl)pyridin-2-yl]amid; cis-4-(2-Oxopyrrolidin-1-yl)cyclohexancarbonsäure[5-(3,5-difluorphenyl)pyridin-2-yl]amid; cis-4-(2-Oxopyrrolidin-1-yl)cyclohexancarbonsäure[5-(3,5-dichlorphenyl)pyridin-2-yl]amid; cis-4-(2-Oxopyrrolidin-1-yl)cyclohexancarbonsäure[5-(3,5-difluorphenyl)-pyrazin-2-yl]amid; 4-(2-Oxoazetidin-1-yl)cyclohexancarbonsäure[5-(3,5-difluorphenyl)pyridin-2-yl]amid; cis-4-(2-Oxopiperidin-1-yl)cyclohexancarbonsäure[5-(3,5-difluorphenyl)pyridin-2-yl]amid; cis-4-(2-Oxopyrrolidin-1-yl)cyclohexancarbonsäure[5-(3-fluor-5-methylphenyl)pyridin-2-yl]amid; cis-4-(2-Oxopyrrolidin-1-yl)cyclohexancarbonsäure[5-(3-chlor-5-methylphenyl)pyridin-2-yl]amid; cis-4-(2-Oxooxazolidin-3-yl)cyclohexancarbonsäure[5-(3,5-difluorphenyl)pyridin-2-yl]amid; trans-4-(2-Oxooxazolidin-3-yl)cyclohexancarbonsäure[5-(3,5-difluorphenyl)pyridin-2-yl]amid; cis-4-(2-Oxooxazolidin-3-yl)cyclohexancarbonsäure[5-(3,5-difluorphenyl)pyrazin-2-yl]amid; cis-4-(2-Oxooxazolidin-3-yl)cyclohexancarbonsäure[1-(3,5-difluorphenyl)-1H-pyrazol-3-yl]amid; trans-4-(2-Oxo[1,3]oxazinan-3-yl)cyclohexancarbonsäure[1-(3,5-difluorphenyl)-1H-pyrazol-4-yl]amid; cis-4-(2-Oxo[1,3]oxazinan-3-yl)cyclohexancarbonsäure[1-(3,5-difluorphenyl)-1H-pyrazol-4-yl]amid; trans-4-(2-Oxo[1,3]oxazinan-3-yl)cyclohexancarbonsäure[1-(2-fluorphenyl)-1H-pyrazol-4-yl]amid; cis-4-(2-Oxo[1,3]oxazinan-3-yl)cyclohexancarbonsäure[1-(2-fluorphenyl)-1H-pyrazol-4-yl]amid; cis-4-(2-Oxo[1,3]oxazinan-3-yl)cyclohexancarbonsäure[5-(3,5-difluorphenyl)-pyrazin-2-yl]amid; cis-4-(2-Oxo-tetrahydropyrimidin-1-yl)cyclohexancarbonsäure[5-(3,5-difluorphenyl)pyridin-2-yl]amid; trans-4-(2-Oxotetrahydropyrimidin-1-yl)cyclohexancarbonsäure[5-(3,5-difluorphenyl)pyridin-2-yl]amid; cis-4-(2-Oxoimidazolidin-1-yl)cyclohexancarbonsäure[5-(3,5-difluorphenyl)pyridin-2-yl]amid; cis-4-(3-Methyl-2-oxotetrahydropyrimidin-1-yl)cyclohexancarbonsäure[5-(3,5-difluorphenyl)pyridin-2-yl]amid; cis-4-(3-Methyl-2-oxoimidazolidin-1-yl)cyclohexancarbonsäure[5-(3,5-difluorphenyl)pyridin-2-yl]amid; trans-4-(3-Methyl-2-oxoimidazolidin-1-yl)cyclohexancarbonsäure[5-(3,5-difluorphenyl)pyridin-2-yl]amid; cis-4-(3-Oxomorpholin-4-yl)cyclohexancarbonsäure[5-(3,5-difluorphenyl)pyridin-2-yl]amid; trans-4-(3-Oxomorpholin-4-yl)cyclohexancarbonsäure[5-(3,5-difluorphenyl)pyridin-2-yl]amid; trans-4-(3-Oxomorpholin-4-yl)cyclohexancarbonsäure[1-(3,5-difluorphenyl)-1H-pyrazol-4-yl]amid; cis-4-(3-Oxomorpholin-4-yl)cyclohexancarbonsäure[1-(3,5-difluorphenyl)-1H-pyrazol-4-yl]amid; trans-4-(3-Oxomorpholin-4-yl)cyclohexancarbonsäure[1-(2-fluorphenyl)-1H-pyrazol-4-yl]amid; cis-4-(3-Oxomorpholin-4-yl)cyclohexancarbonsäure[1-(2-fluorphenyl)-1 H-pyrazol-4-yl]amid; cis-4-(3-Oxomorpholin-4-yl)cyclohexancarbonsäure[5-(3,5-difluorphenyl)pyrazin-2-yl]amid; und cis-4-(3-Oxomorpholin-4-yl)cyclohexancarbonsäure[5-(3,5-difluorphenyl)pyrimidin-2-yl]amid; cis-4-(2-Oxopyrrolidin-1-yl)cyclohexancarbonsäure[5-(3,5-difluorphenyl)pyrimidin-2-yl]amid; cis-4-(2-Oxooxazolidin-3-yl)cyclohexancarbonsäure[5-(3,5-difluorphenyl)pyrimidin-2-yl]amid und cis-4-(3-Methyl-2-oxoimidazolidin-1-yl)cyclohexancarbonsäure[5-(3,5-difluorphenyl)pyrimidin-2-yl]amid.

22. Pharmazeutische Zusammensetzung, die eine therapeutisch wirksame Menge der Verbindung nach einem beliebigen der Ansprüche 1-21 und einen pharmazeutisch akzeptablen Träger umfasst.

23. Verwendung einer Verbindung nach einem beliebigen der Ansprüche 1-21 zur Herstellung eines Medikaments, das von Nutzen bei der Behandlung des ADHS ist.

24. Verwendung einer Verbindung nach einem beliebigen der Ansprüche 1-21 zur Herstellung eines Medikaments, das von Nutzen bei der Behandlung einer kognitiven Störung ist.

25. Verwendung einer Verbindung nach einem beliebigen der Ansprüche 1-21 zur Herstellung eines Medikaments, das von Nutzen bei der Behandlung der Schizophrenie ist.

## Revendications

1. Composé de formule I : 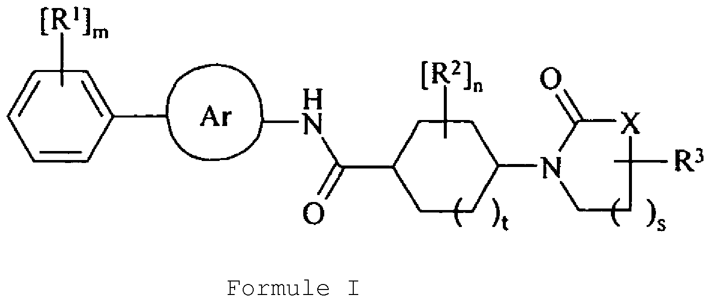 dans laquelle R¹ est un groupement alkyle en C₁-C₇, perfluoroalkyle en C₁-C₇, alcoxy en C₁-C₇ ou un halogène ;
dans laquelle R² est un groupement alkyle en C₁-C₇ ;
dans laquelle R³ est H, un groupement alkyle en C₁-C₇, alcoxy en C₁-C₇, -(CH₂)ᵤOH, -N(R⁴)C(O)alkyle en C₁-C₇ ou -N(R⁴)C(O) alcoxy en C₁-C₇ ;
dans laquelle R⁴ est H ou un groupement alkyle en C₁-C₇ ;
dans laquelle X est -CH₂-, -O-, -CH₂O-, -NR⁵- ou -CH₂NR⁵- ;
dans laquelle R⁵ est H, un groupement alkyle en C₁-C₇, phényle ou pyridyle, où les groupements phényle et pyridyle sont éventuellement substitués par un groupement alkyle en C₁-C₇, perfluoroalkyle en C₁-C₇, alcoxy en C₁-C₇ ou un halogène ;
dans laquelle Ar est un radical aromatique divalent choisi dans le groupe constitué des groupements phényle, pyridyle, pyrimidine, pyridazine, pyrazine, imidazole, pyrazole , triazole, oxazole et isoxazole ;
dans laquelle chaque m et chaque n sont indépendamment un entier de 0 à 5 inclus ;
dans laquelle u est 0 ou 1 ; et
dans laquelle chaque t et chaque s est indépendamment un entier de 0 à 2 inclus ; ou un sel de ce composé acceptable au plan pharmaceutique.

2. Composé selon la revendication 1, dans lequel Ar est un groupement phényle.

3. Composé selon la revendication 1, dans lequel Ar est la pyridine ou la pyrimidine.

4. Composé selon la revendication 1, dans lequel Ar est la pyridazine, la pyrazine ou le triazole.

5. Composé selon la revendication 1, dans lequel Ar est l'imidazole, le pyrazole, l'oxyzole ou l'isoxazole.

6. Composé selon la revendication 1, dans lequel t est 1 et le radical cyclohexane est en configuration sis.

7. Composé selon la revendication 1, dans lequel t est 1 et le radical cyclohexane est en configuration trans.

8. Composé selon la revendication 1, dans lequel X est -CH₂-.

9. Composé selon la revendication 1, dans lequel X est -O-.

10. Composé selon la revendication 1, dans lequel X est -CH₂O-.

11. Composé selon la revendication 1, dans lequel X est -NR⁵- et R⁵ est H ou un groupement alkyle en C₁-C₄.

12. Composé selon la revendication 1, dans lequel X est -CH₂NR₅- et R⁵ est H ou un groupement alkyle en C₁-C₄.

13. Composé selon la revendication 1, dans lequel R¹ est un groupement alcoxy en C₁-C₄ ou perfluoroalkyle en C₁-C₄ ; et m est 0, 1 ou 2.

14. Composé selon la revendication 1, dans lequel R¹ est un groupement alkyle en C₁-C₄, F ou Cl ; et m est 0, 1 ou 2.

15. Composé selon la revendication 1, dans lequel n est 0.

16. Composé selon la revendication 1, dans lequel s est 0 ou 1.

17. Composé selon la revendication 1, dans lequel R³ est H.

18. Composé selon la revendication 1, dans lequel R³ est un groupement alkyle en C₁-C₄, alcoxy en C₁-C₄ ou -(CH₂)ᵤOH ; et u est 0.

19. Composé selon la revendication 1, dans lequel R³ est un groupement alkyle en C₁-C₄, alcoxy en C₁-C₄ ou -(CH₂)ᵤOH ; et u est 1.

20. Composé selon la revendication 1, dans lequel R³ est un groupement -N(R⁴)C(O)alkyle en C₁-C₄ ou -N(R⁴)C(O)alcoxy en C₁-C₄ ; et R⁴ est H ou un groupement alkyle en C₁-C₄.

21. Composé selon la revendication 1, dans lequel le composé est choisi dans le groupe constitué des suivants :
[5-(3,5-difluoro-phényl)pyridin-2-yl]amide de l'acide trans-4-(2-oxo-pyrrolidin-1-yl)cyclohexane-carboxylique ;
[5-(3,5-difluoro-phényl)pyridin-2-yl]amide de l'acide cis-4-(2-oxo-pyrrolidin-1-yl)cyclohexane-carboxylique ;
[5-(3,5-dichloro-phényl)pyridin-2-yl]amide de l'acide cis-4-(2-oxo-pyrrolidin-1-yl)cyclohexane-carboxylique ;
[5-(3,5-difluoro-phényl)pyrazin-2-yl]amide de l'acide cis-4-(2-oxo-pyrrolidin-1-yl)cyclohexane-carboxylique ;
[5-(3,5-difluoro-phényl)pyridin-2-yl]amide de l'acide 4-(2-oxo-azétidin-1-yl)cyclohexanecarboxylique ;
[5-(3,5-difluoro-phényl)pyridin-2-yl]amide de l'acide cis-4-(2-oxo-pipéridin-1-yl)cyclohexane-carboxylique ;
[5-(3-fluoro-5-méthyl-phényl)pyridin-2-yl]amide de l'acide cis-4-(2-oxo-pyrrolidin-1-yl)cyclohexane-carboxylique ;
[5-(3-chloro-5-méthyl-phényl)pyridin-2-yl]amide de l'acide cis-4-(2-oxo-pyrrolidin-1-yl)cyclohexane-carboxylique ;
[5-(3,5-difluoro-phényl)pyridin-2-yl]amide de l'acide cis-4-(2-oxo-oxazolidin-3-yl)cyclohexane-carboxylique ;
[5-(3,5-difluoro-phényl)pyridin-2-yl]amide de l'acide trans-4-(2-oxo-oxazolidin-3-yl)cyclohexane carboxylique ;
[5-(3,5-difluoro-phényl)pyrazin-2-yl]amide de l'acide cis-4-(2-oxo-oxazolidin-3-yl)-cyclohexane-carboxylique ;
[1-(3,5-difluoro-phényl)-1H-pyrazol-3-yl]amide de l'acide cis-4-(2-oxo-oxazolidin-3-yl)cyclohexane carboxylique ;
[1-(3,5-difluoro-phényl)-1H-pyrazol-4-yl]amide de l'acide trans-4-(2-oxo-[1,3]oxazinan-3-yl)cyclo-hexanecarboxylique ;
[1-(3,5-difluoro-phényl)-1H-pyrazol-4-yl]amide de l'acide cis-4-(2-oxo-[1,3]oxazinan-3-yl)cyclo-hexanecarboxylique ;
[1-(2-fluoro-phényl)-1H-pyrazol-4-yl]amide de l'acide trans-4-(2-oxo-[1,3]oxazinan-3-yl)cyclohexane-carboxylique ;
[1-(2-fluoro-phényl)-1H-pyrazol-4-yl]amide de l'acide cis-4-(2-oxo-[1,3]oxazinan-3-yl)cyclohexane-carboxylique ;
[5-(3,5-difluoro-phényl)pyrazin-2-yl]amide de l'acide cis-4-(2-oxo-[1,3]oxazinan-3-yl)cyclohexane-carboxylique ;
[5-(3,5-difluoro-phényl)pyridin-2-yl]amide de l'acide cis-4-(2-oxo-tétrahydro-pyrimidin-1-yl)cyclo-hexanecarboxylique ;
[5-(3,5-difluoro-phényl)pyridin-2-yl]amide de l'acide trans-4-(2-oxo-tétrahydro-pyrimidin-1-yl)cyclo-hexanecarboxylique ;
[5-(3,5-difluoro-phényl)pyridin-2-yl]amide de l'acide cis-4-(2-oxo-imidazolidin-1-yl)cyclohexane-carboxylique ;
[5-(3,5-difluoro-phényl)pyridin-2-yl]amide de l'acide cis-4-(3-méthyl-2-oxo-tétrahydro-pyrimidin-1-yl)cyclohexane-caxboxylique ;
[5-(3,5-difluoro-phényl)pyridin-2-yl]amide de l'acide cis-4-{3-éthyl-2-oxo-imidazolidin-2-yl)cyclohexanecarboxylique ;
[5-(3,5-difluoro-phényl)pyridin-2-yl]amide de l'acide trans-4-méthyl-2-oxo-imidazolidin-1-yl)cyclohexanecarboxylique ;
[5-(3,5-difluoro-phényl)pyridin-2-yl]amide de l'acide cis-4-(3-oxo-morpholin-4-yl)cyclohexanecarboxylique ;
[5-(3,5-difluoro-phényl)pyridin-2-yl]amide de l'acide trans-4-(3-oxo-morpholin-4-yl)cyclohexanecarboxylique ;
[1-(3,5-difluoro-phényl)-1H-pyrazol-4-yl]amide de l'acide trans-4-(3-oxo-morpholin-4-yl)cydohexanecarboxylique ;
[1-(3,5-difluoro-phényl)-1H-pyrazol-4-yl]amide de l'acide cis-4-(3-oxo-morpholin-4-yl)cyclohexanecarboxylique ;
[1-(2-fluoro-phényl)-1H-pyrazol-4-yl]amide de l'acide trans-4-(3-oxo-morpholin-4-yl)cyclohexanecarboxylique ;
[1-(2-fluoro-phényl)-1H-pyrazol-4-yl}amide de l'acide cis-4-(3-oxo-morpholin-4-yl)cyclohexanecarboxylique ;
[5-(3,5-difluoro-phényl)pyrazin-2-yl]amide de l'acide cis-4-(3-oxo-morpholin-4-yl)cyclohexanecarboxylique ; et
[5-(3,5-difluoro-phényl)pyrimidin-2-yl]amide de l'acide cis-4-(3-oxo-morpholin-4-yl)cyclohexanecarboxylique ;
[5-(3,5-difluoro-phényl)pyrimidin-2-yl]amide de l'acide cis-4-(2-oxo-pyrrolidin-1-yl)cyclohexanecarboxylique ;
[5-(3,5-difluoro-phényl)pyrimidin-2-yl]amide de l'acide cis-4-(2-oxo-oxazolidin-3-yl)cyclohexanecarboxylique ; et
[5-(3,5-difluoro-phényl)pyrimidin-2-yl)amide de l'acide cis-4-(3-méthyl-2-oxo-imidazolidin-1-yl)cyclohexanecarboxylique.

22. Composition pharmaceutique comprenant une quantité efficace au plan thérapeutique du composé de l'une quelconque des revendications 1 à 21 et un véhicule acceptable au plan pharmaceutique.

23. Utilisation d'un composé selon l'une quelconque des revendications 1 à 21 pour la fabrication d'un médicament utile pour le traitement de l'ADHD.

24. Utilisation d'un composé selon l'une quelconque des revendications 1 à 21 pour la fabrication d'un médicament utile pour le traitement d'un trouble cognitif.

25. Utilisation d'un composé selon l'une quelconque des revendications 1 à 21 pour la fabrication d'un médicament utile pour le traitement de la schizophrénie.
